# EUROPEAN PATENT SPECIFICATION

(11) **EP 1 121 441 B1**
(45) Date of publication and mention of the grant of the patent: **13.12.2006**
(21) Application number: 99951481.3
(22) Date of filing: 14.10.1999
(51) Int. Cl.: C12N 15/34, C12N 15/62, C12N 15/861, C07K 14/075, C07K 14/47, A61P 35/00, A61K 48/00

(54) **SELECTIVELY REPLICATING VIRAL VECTORS**
SELEKTIV-REPLIZIERENDE VIRALE VECTOREN
VECTEURS VIRAUX A REPLICATION SELECTIVE

(30) Priority: 15.10.1998 US 172686
(43) Date of publication of application: 08.08.2001
(73) Proprietor: CANJI, Inc., San Diego, CA 92121 (US)
(72) Inventor: RAMACHANDRA, Muralidhara, San Diego, CA 92128 (US); SHABRAM, Paul, W., Olivenhain, CA 92024 (US)
(74) Representative: UEXKÜLL & STOLBERG
(86) International application number: PCT/US1999/021452
(87) International publication number: WO 2000/022137

(56) References cited:
- WO-A-94/18992
- WO-A-98/13508
- WO-A-98/21228
- WO-A-98/39464
- ZHANG W ET AL.: "The requirement of the carboxyl terminus of p53 for DNA binding and transcriptional activation depends on the specific p53 binding DNA element" ONCOGENE, vol. 9, no. 9, September 1994 (1994-09), pages 2513-2521, XP000891651
- DENNLER S ET AL.: "Direct binding of Smad3 and Smad4 to critical TGFbeta-inducible elements in the promoter of human plasminogen activator inhibitor-type 1 gene" THE EMBO JOURNAL, vol. 17, no. 11, 1 June 1998 (1998-06-01), pages 3091-3100, XP002134419
- ELSING A ET AL.: "The adenovirus E3/10.4K-14.5K proteins down-modulate the apoptosis receptor Fas/Apo-1 by inducing its internalization" PROC. NATL. ACAD. SCI. USA, vol. 95, no. 17, 18 August 1998 (1998-08-18), pages 10072-10077, XP002134420
- KIRN D H ET AL.: "Replicating viruses as selective cancer therapeutics" MOLECULAR MEDICINE TODAY, vol. 2, December 1996 (1996-12), pages 519-527, XP000891648
- MILLER R ET AL.: "Towards the use of replicative adenoviral vectors for cancer gene therapy" GENE THERAPY, vol. 3, no. 7, July 1996 (1996-07), pages 557-559, XP000891689
- MORI N. ET AL: 'Repression of Transcription from the Human T-Cell Leukemia Virus Type I Long terminal Repeat and Cellular Gene Promoters by Wild-Type p53' BLOOD, AMERICAN SOCIETY OF HEMATOLOGY vol. 90, no. 12, 15 December 1997, pages 4924 - 4932

## Description

### BACKGROUND OF THE INVENTION

Recombinant adenoviruses are currently used for the delivery of therapeutic transgenes in a variety of therapeutic regimens. However, the broad range of infectivity of these vector systems has raised concerns that the expression of the virus in non-tumor cells might cause collateral damage to non-neoplastic cells. Consequently, a broad range of targeting systems have been developed to preferentially express the transgene in a given cell type. Tissue specific and tumor specific promoters have been employed to preferentially replicate the vector in certain cell types. For example, International Patent Application No. PCT/US96/10838 published January 16, 1997 (International Publication No. WO97/01358) describes the use of vectors which replicate in a specific host cell by the use of prostate specific promoter elements driving the E1, E2 or E4 functions, optionally containing a cytotoxic transgene expression cassette. In particular, this publication describes a construct where prostate specific enhancer controls expression of E1 and has a expression cassette comprising the CMV-promoter driving expression of the cytosine deaminase gene which is inserted into the E3 region These vectors are replication competent and are capable of packaging into intact virions in a particular cell type.

An alternative approach to the use of tumor specific promoters to drive viral replication is to employ specific deletions in the adenoviral E1b 55K protein coding sequence. Recombinant adenoviruses which contain defects in the nucleotide sequence encoding E1b 55K are described in United States Patent No. 5,677,178 issued October 14, 1997. However, these tissue or tumor specific control elements have been observed to be "leaky", i.e. permitting replication in cell types other than the preferred target cells.

Alternative to this type of selectively replicating vector is the employment of a replication deficient adenoviral vector containing extensive elimination of E1 function. In particular, vectors containing elimination of E1, E2 E3 and partial E4 deletions have been employed to delivery exogenous transgenes. Such vectors have been employed to deliver the p53 gene to target cells. It has been demonstrated that the expression of an exogenously administered wild type p53 in a p53 deficient (p53 mutated or p53 null) tumor cell is capable of inducing p53 mediated apoptosis in the tumor cell. Such viral vectors for the delivery of p53 are currently under development Schering Corporation and Introgen Corporation. Again these vectors have demonstrated acceptable toxicology profiles and therapeutic efficacy for human therapeutic applications and are in Phase II clinical trials in man for the treatment of p53 related malignancies.

Replication deficient and selectively replicating vectors have, at least in theory, design drawbacks which are of concern to clinicians. Because replication deficient vectors will not propagate uncontrollably in the patient, they theoretically have a more appealing safety profile. However, as effective tumor elimination requires the infection of the substantial majority of the tumor cells being infected, a substantial molar excess of vector is commonly used to insure therapeutic effectiveness. Selectively replicating vectors are viewed as being more of an issue from a safety perspective because of their ability to replicate and potentially mutate to form fully replication competent vectors in the patient. However, by maintaining the natural ability to the virus to propagate under particular conditions enables these vectors to spread to surrounding tumor cells. Since the vectors themselves are able to replicate, a lower initial dose of such vectors is required. This is favorable from an immunological perspective as well as for economic reasons in the manufacture of such agents. Therefore, there is a need in the art for a selectively replicating vector which addresses the perceived safety problems while providing the increased therapeutic index.

WO 9839464 is related to adenovirus vectors, specific for target cells and methods of use of such viruses. Adenoviral replication can be restricted to target cells by transcriptional control with cell-specific heterologous transcriptional regulatory elements so that the adenovirus vectors can replicate in the target cells and provide cell-specific cytotoxicity, particularly to neoplastic cells.

WO 9821228 is related to fusion proteins of an E2F polypeptide with an RB polypeptide, which are efficient as repressors. Also suggested is the tissue-specific expression of these constructs. Adenovirus is suggested as a preferred vector system.

Elsing et al. ((1998) PNAS 95 (17) 10072 - 10077) describe the observation that disruption of ORF 14.5K of adenovirus rescues expression of FAS/APO-1 on the surface of adenovirus infected cells.

WO 9418992 is related to methods and compositions for treating neoplastic conditions by viral-based therapy. Mutant virus lacking viral proteins which bind and/or inactivate p53 or RB are administered to a patient having a neoplasm which comprises cells lacking p53 and/or RB functions. This is intended to preferentially generate a replication phenotype in neoplastic cells, resulting in the preferential killing of the neoplastic cells.

Mori et al. ( (1997) Blood 90 (12) 4924 - 4932) discloses that expression of certain genes from the HTLV-1 virus is repressed by functional p53 proteins. In cells where p53 function is abolished a high expression of viral genes (regulated by the Tax gene product) is observed, leading to an establishment of productive viral infection.

The present invention solves these problems by providing a selectively replicating adenoviral vector containing a pathway targeted pathway-responsive promoter driving expression of a repressor of viral replication such that the vector replicates preferentially in cells defective in the pathway. The present invention also provides pharmaceutical formulations comprising such vectors. The present invention also provides methods of eliminating pathway defective cells from a population of normal cells by using such vectors.

### SUMMARY OF THE INVENTION

The present invention provides recombinant viruses which replicate the viral genome selectively in response to the intracellular conditions of the target cell through the use a pathway-responsive promoter driving expression of an inhibitor of viral replication which substantially inhibits viral replication in the host cell based on the phenotypic or genotypic of the infected cell. In the target cell, the promoter element of the pathway-responsive promoter is *inactive* and thus the virus is permitted to replicate. This results in: (1) killing the cells by natural lytic nature of the virus, and/or (2) provides a therapeutic dose of a transgene product (amplified in comparison to replication incompetent vectors) to the target cell, and (3) producing a localized concentration of the virus facilitating the infection of surrounding target cells to the recombinant virus. The invention further provides therapeutic and diagnostic methods of use of the vectors, pharmaceutical formulations comprising the vectors, methods of making the vectors and transformed cells comprising the vectors.

### BRIEF DESCRIPTION OF THE FIGURES

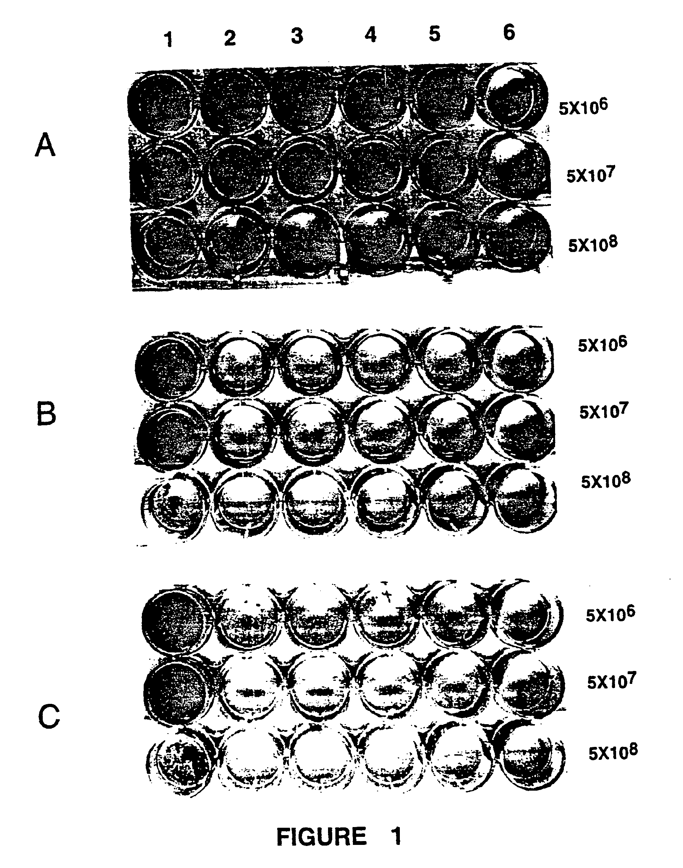

Figure 1. Results of CPE assay to determine cytopathic effect of recombinant adenovirus vectors encoding E2F-Rb fusion coding sequence under the control of either TGF-β pathway-responsive promoter (PAI or SRE) or p53 pathway-responsive promoter (RGC or p53CON) were generated. In addition, the gene encoding green fluorescent protein was also incorporated in the vectors as a reporter. Panel A represents MRC-9 cells, Panel P represents the Hep3B cells, and Panel C represents WIDR cells. Lane 1: replication deficient (E1 deleted) recombinant adenovirus expressing the green fluorescent protein (GFP); Lane 2: PAI-Ad; Lane 3: SRE-Ad; Lane 4: RGC-Ad; Lane 5 p53CON-Ad. Particle concentrations are as indicated at the right of the Figure.

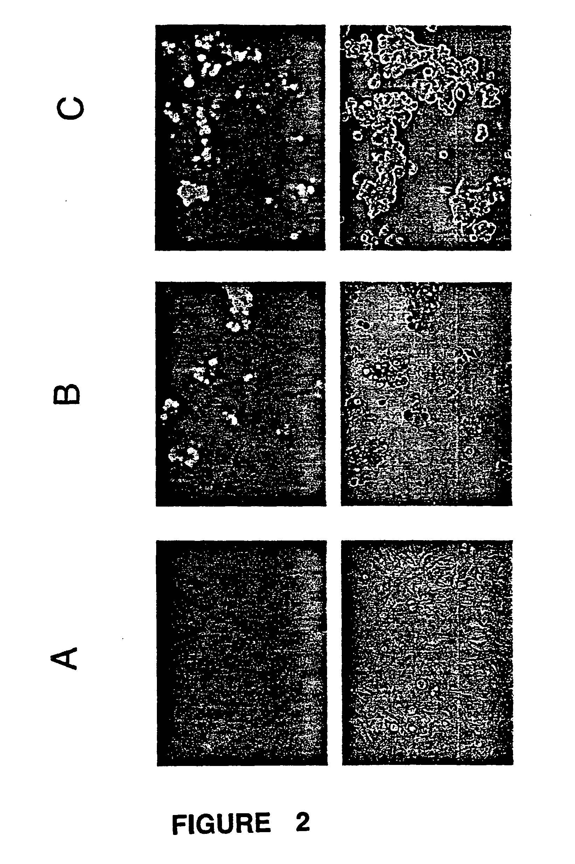

Figure 2. Results of fluororesence microscopy (upper panels) illustrating GFP expression with pathway targeted vectors. Panel A represents the GFCB control vector, Panel B the SRE-Ad vector and Panel C the p53CON-Ad vector. Infection was at 5 x 10⁵ particles per milliliter.

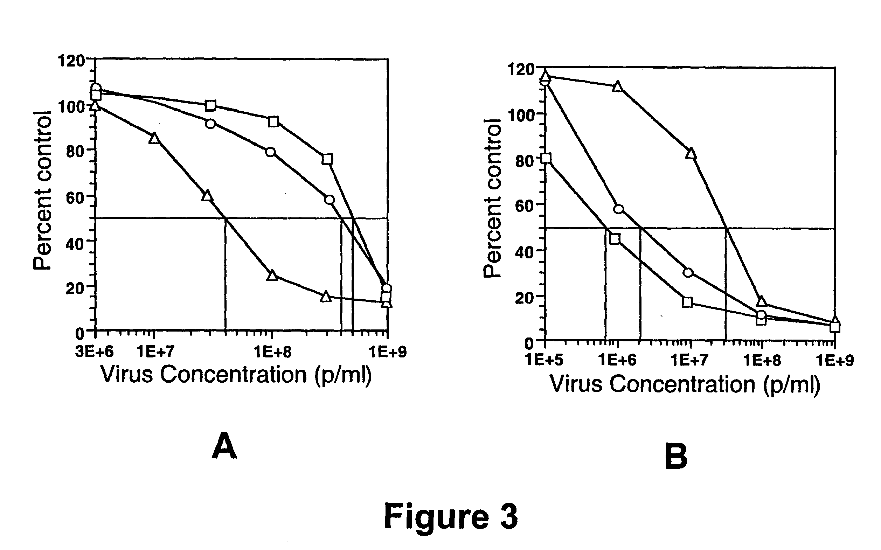

Figure 3. Results of infection of normal bronchial epithelial cells (Panel A) and C33A cells (Panel B) with the recombinant viruses U3EE (squares), T1LT (circles) and L9IU (triangles). The vertical axes represent the percent of uninfected controls and the horizontal axes represent the viral dose in particles per milliliter. The experiments were performed by exposing a culture of each cells to six different concentrations of virus from 10⁵ to 10⁹ viral particles per milliliter. The cells were exposed to the virus for a period of one hour, the excess virus washed and the percent of viable cells at six days following infection was determined the MTS assay (Promega, Madison WI) in substantial accordance with the manufacturer's instructions. The horizontal line represents the level at which 50% of the cells remained viable. The intersection of the curves generated by the data and the horizontal dotted line is a measure of the ED₅₀ of the virus.

### DETAILED DESCRIPTION OF THE INVENTION

The present invention provides a selectively replicating recombinant viral vector comprising a pathway-responsive promoter operably linked to a repressor of viral replication, wherein the vector selectively replicates in neoplastic cells possessing a defect in the pathway to which the promoter is responsive.

The term "selectively replicating" refers to a vector capable of preferential replication in a cell in one phenotypic state relative to another phenotypic state. Examples of different phenotypic states would include the p53 pathway defective versus a normal cell of a given cell type. A virus which exhibits preferential replication indicates that the virus replicates at least five times as efficiently in the target cell type relative to a normal (control) cell of the same type at a given dosage level. In order to determine if a virus is truly selective, it is necessary to evaluate the ability of the virus to replicate in target cells as compared to normal cells of the same cell type in regard to a number of factors.

It is preferred that one compare the ability of a given vector to replicate in a target cell containing the condition to be targeted and normal cell of the same type which does not possess this condition. For example, the first step following viral infection is to induce the cell to enter the cell cycle because factors essential in for maximal viral replication efficiency are present only in S-phase. However, if one were attempting to evaluate the selectivity of a vector for selectivity in tumor cells, evaluation of the ability to replicate in a tumor cell relative to another cell which has already entered the cell cycle (such as an immortalized or transformed cell line), the selectivity of the virus for tumor cells will, to some degree, be obscured. Additionally, it has been observed that different cell types possess widely differing infectivities to a given virus. Although some viruses such as adenoviruses possess broad tissue tropism, other viruses are more restricted in the type of cells which they infect. By attempting to evaluate the performance of a given vector in cells of widely differing infectivities, it will be difficult to assess whether a lack of effect is due to the performance of the vector within the cell or merely the failure of the virus to infect the cell at all. By evaluating selectivity in target and normal cells of a given type, one minimizes this infectivity effect.

The temporal nature of the viral life cycle must also be considered. For example, even a wild-type vector may appear to possess selectivity in tumor cells relative to normal cells early after infection because the cell is already cycling. However this apparent selectivity diminishes over time once the virus has stimulated the cell cycle. Consequently, the time at which selectivity is evaluated following infection must be sufficient to avoid this initial replication lag in normal cells. Although this will vary with the type of virus being employed, this initial lag can readily be determined by one of skill in the art.

The effect of dosage must also be considered in the determination of whether a given recombinant adenovirus is demonstrating a selective effect in the target cell type. For example, if one is targeting the elimination of tumor cells and measuring the effect by cytotoxicity, a virus may be made to appear to have selective cytotoxicity by differing dosages. It has been observed that a sufficiently high dose almost any virus, regardless of the degree to which its genome has been modified, will be cytotoxic due simply to the effects of the presence of the viral proteins (such as hexon in the case of adenovirus) which are known to be cytotoxic. Similarly, even though the scientific literature may refer to a virus as "replication defective" (suggesting that the virus is absolutely incapable of replication in the absence of a cell line capable of complementing the viral defect), such viruses are more accurately described as "attenuated for replication." For example, adenoviruses containing a deletion of the entire E1 region which are frequently referred to as "replication deficient" or "replication defective" will replicate to some degree, particularly in cycling or rapidly dividing cells. As Mulligan observed (1990, Science 260:926-932):

Although the expression of the E1 region has been shown to affect the expression of other viral gene products necessary for replication *(citing* Horwitz, M. in Virology, B.N. Fields Ed. (Raven, New York, 1990) Chapter 60)), the required of E1 gene expression for viral replication does not appear to be absolute. The early characterization of E1-deficient viruses demonstrate that at high multiplicities of infection, the E1 region was dispensable for replication (citing Jones and Shenk (1979) PNAS(USA) 76(8):3665-3669).

Consequently, the effect of viral dose cannot be ignored when determining whether or not a virus is replicating in a selective manner.

One means to evaluate the replication selectivity of a virus for the target cells is to use evaluate the virus's "selectivity index" as follows. The commonly used parameter ED₅₀ (which is defined as the dose sufficient to induce cell death in 50% of the cells) provides an appropriate basis of comparison. The ED₅₀ of a virus can readily be determined by typical in vitro dose escalation experiments. In order to ensure the most consistent basis of comparison, the ED₅₀ is most appropriately expressed relative to a viral control to minimize the effects of variations of infectivity between the cell types being compared and any assay variations. Consequently the unitless ratio: ED₅₀(virus)/ED₅₀(control) is used to express the relative toxicity of the virus in the cell and will be referred to as the "relative toxicity index" or "RTI." The "selectivity index" of a given virus is expressed by the ratio: RTI(target cells)/RTI(normal cells). Selectively replicating vectors will possess a selectivity index of at least 10, but preferably 50, 100 or greater.

For example, the selectively replicating adenoviral vectors U3EE and T1LT are designed to achieve selective replication and killing of tumor cells having p53 pathway defects. The U3EE is prepared in substantial accordance with the teaching of Examples herein. Briefly, the U3EE virus contains a first expression cassette comprising a p53 response element (p53 CON) driving expression of the E2F-Rb fusion protein. The E2F-Rb fusion protein is a potent inhibitor of adenoviral E2 promoter activity and its presence in the cell will effectively suppress viral replication. The p53 response element is active in response to the presence of a functional p53 pathway. Consequently, in normal cells where the p53 pathway is intact, the U3EE virus will express the E2F-Rb fusion protein and the virus will not replicate. However, in cells having p53 pathway defect (the majority of tumor cells), the p53CON response element is not active and thus there is no repression of viral replication. The U3EE vector also contains an expression cassette comprising the MLP promoter driving expression of the Ad5 E3-10.5K pro-apoptotic gene. The use of the temporal promoters (such as the MLP promoter) is preferred when employing pro-apoptotic genes because one wishes to facilitate replication of viral DNA within the target cell prior to activating the pro-apoptotic signal. The MLP promoter is activated approximately seven hours post-infection following onset if replication of the U3EE genome thus inducing the activity of the E3-10.5 K protein. The T1LT adenoviral vector is essentially the same as the U3EE vector except that it contains an additional deletion in the E1a region to removes amino acids 4-25 of the 243R and 289R adenoviral E1a proteins. This deletion disrupts the ability of the p300 protein to bind to these E1a proteins.

The U3EE and T1LT viruses were evaluated for their ability to replicate in and kill normal human brochial epitelial cells (NHBE) and C33A (an epithelial tumor cell line having a p53 defective pathway) using the L9IU vector as the control. The results of these experiments are presented in Figure 3 of the accompanying drawings. The following table summarizes the data presented:

| **Table 1. Summary of RTI and Selectivity Indices of Viruses** | | | |
|---|---|---|---|
| **Virus** | **RTI (normal cells)** | **RTI (tumor cells)** | **Selectivity Index** |
| **L9IU** | 1.0 | 1.0 | 1.0 |
| **U3EE** | 12.5 | 0.0233 | 536 |
| **T1LT** | 10 | 0.066 | 152 |

As can be seen from the data presented, the U3EE and T1LT viruses possesses high selectivity for tumor cells. As previously discussed, the L9IU virus possesses a slight replication advantage in the cycling tumor cells relative to the quiescent normal cells. By comparing the ratios of ED₅₀(virus)/ED₅₀(control) in each of the cell types prior to calculating the selectivity index, the effects of such variations is minimized.

The term "recombinant" refers to a genome which has been modified through conventional recombinant DNA techniques.

The term "virus" refers to any of the obligate intracellular parasites having no protein-synthesizing or energy-generating mechanism. The viral genome may be RNA or DNA contained with a coated structure of protein of a lipid membrane. Examples of viruses useful in the practice of the present invention include baculoviridiae, parvoviridiae, picornoviridiae, herepesviridiae, poxviridiae, adenoviridiae, picotmaviridiae. The term recombinant virus includes chimeric (or even multimeric) viruses, i.e. vectors constructed using complementary coding sequences from more than one viral subtype. See, e.g. Feng, *et al.* Nature Biotechnology 15:866-870

The term "adenovirus" is synonomous with the term "adenoviral vector" and refers to viruses of the genus adenoviridiae. The term adenoviridiae refers collectively to animal adenoviruses of the genus mastadenovirus including but no limited to human, bovine, ovine, equine, canine, porcine, murine and simian adenovirus subgenera. In particular, human adenoviruses includes the A-F sugenera as well as the individual serotypes thereof the individual serotypes and A-F subgenera including but not limited to human adenovirus types 1, 2, 3, 4, 4a, 5, 6, 7, 8, 9, 10, 11 (Ad11A and Ad 11P), 12, 13,14,15,16,17,18,19, 19a, 20, 21, 22, 23, 24, 25, 26, 27, 28, 29, 30, 31, 32, 33, 34, 34a, 35, 35p, 36, 37, 38, 39, 40, 41, 42, 43, 44, 45, 46, 47, 48, and 91. The term bovine adenoviruses includes but is not limited to bovine adenovirus types 1,2,3,4,7, and 10. The term canine adenoviruses includes but is not limited to canine types 1 (strains CLL, Glaxo, RI261, Utrect, Toronto 26-61) and 2. The term equine adenoviruses includes but is not limited to equine types 1 and 2. The term porcine adenoviruses includes but is not limited to porcine types 3 and 4. In the preferred practice of the invention, the adenovirus is derived from the human adenovirus serotypes 2 or 5.

The term "pathway-responsive promoter" refers to DNA sequences that bind a certain protein and cause nearby genes to respond transcriptionally to the binding of the protein in normal cells. Such promoters may be generated by incorporating response elements which are sequences to which transcription factors bind. Such responses are generally inductive, though there are several cases where increasing protein levels decrease transcription. Pathway-responsive promoters may be naturally occuring or synthetic. Pathway-responsive promoters are typically constructed in reference to the pathway or a functional protein which is targeted. For example, a naturally occurring p53 pathway-responsive promoter would include transcriptional control elements activated by the presence of functional p53 such as the p21 or bax promoter. Alternatively, synthetic promoters containing p53 binding sites upstream of a minimal promoter (e.g. the SV40 TATA box region) may be employed to create a synthetic pathway-responsive promoter. Synthetic pathway-responsive promoters are generally constructed from one or more copies of a sequence that matches a consensus binding motif. Such consensus DNA binding motifs can readily be determined. Such consensus sequences are generally arranged as a direct or head-to-tail repeat separated by a few base pairs. Elements that include head-to-head repeats (eg. AGGTCATGACCT) are called palindromes or inverted repeats and those with tail-to-tail repeats are called everted repeats.

Examples of pathway-responsive promoters useful in the practice of the present invention include synthetic insulin pathway-responsive promoters containing the consensus insulin binding sequence (Jacob, *et al.* (1995). J. Biol. Chem. 270:27773-27779), the cytokine pathway-responsive promoter, the glucocorticoid pathway-responsive promoter (Lange, *et al.*(1992) J Biol Chem 267:15673-80), IL1 and IL6 pathway-responsive promoters (Won K.-A and Baumann H. (1990) Mol.Cell.Biol. 10: 3965-3978), T3 pathway-responsive promoters, thyroid hormone pathway-responsive promoters containing the consensus motif: 5' AGGTCA 3.', the TPA pathway-responsive promoters (TREs), TGF-β pathway-responsive promoters (as described in Grotendorst, *et al.*(1996) Cell Growth and Differentiation 7: 469-480). Examples of other pathway-responsive promoters are well known in the art and can be identified in the Database of Transcription Regulatory Regions on Eukaryotic Genomes accessible through the internet at http://www.eimb.rssi.ru/TRRD.

In the preferred practice of the invention as exemplified herein, the vector comprises a synthetic TGF-β pathway-responsive promoter active in the presence of a functional TGF-β pathway such as the promoter containing SRE and PAI-RE response elements. PAI-RE refers to a synthetic TGF-β response element comprising sequences responsive to TGF-β signal isolated from the plasminogen activator-I promoter region. The construction of PAI-RE is described in Example 3 herein. The PAI-RE pathway-responsive promoter may be isolated as a 749 base pair fragment isolatable from the plasmid p8001uc (as described in Zonneveld, *et al.* (1988) PNAS 85:5525-5529 and available from GenBank under accession number J03836). SRE refers to a synthetic TGF-β response element comprising a repeat of 4 of the Smad-4 DNA binding sequences (GTCTAGAC as described in Zawel, *et al.* (1988) Mol. Cell 1:611-617 The construction of SRE is described in Example 3 herein. The SRE response element may be generated by annealing complimentary oligonucleotides encoding the Smad-4 binding sequences and cloning in plasmid pGL#3 - promoter luciferase vector (commercially availabe from ProMega).

Similarly, a "p53 pathway-responsive promoter" refers to a transcriptional control element active in the presence of a functional p53 pathway. The p53 pathway-responsive promoter may be a naturally occurring transcriptional control region active in the presence of a functional p53 pathway such as the p21 or mdm2 promoter. Alternatively, the p53 pathway-responsive promoter may be a synthetic transcriptional control region active in the presence of a functional p53 pathway such as the SRE and PAI-RE pathway-responsive promoters. p53-CON describes a p53 pathway-responsive promoter containing a synthetic p53 response element constructed by insertion of two synthetic p53 consensus DNA binding sequences (as described in Funk, *et al.* (1992) Mol. Cell Biol. 12:2866-2871) upstream of the SV40 TATA box. The construction of the p53-CON pathway-responsive promoter is described in Example 3 herein. RGC refers to a synthetic p53 pathway-responsive promoter using a single p53 binding domain identified in the ribosomal gene cluster. Kern, et al. (1991) Science 252:1708-1711 and Park, et al (1996) Molecular Carcinogenesis 16:101-108. p53CON and RGC response elements can be constructed by annealing complimentary oligonucleotides and p53 responsive promoters can be constructed (as described more fully in Example 3) by cloning in plasmid pGL3-promoter luciferase vector (commercially available from ProMega)

The term "target cell" refers to a cell of a given phenotypic state which is desired to be treated by administration of a therapeutic transgene. By the use of various pathway responsive promoter elements, one can target the expression of the virus to any given cell with an intact pathway. For example the repressor of viral replication may be expressed in a neoplastic target cell through the use of TGF-beta or p53 pathway responsive promoters. Similarly, the repressor of viral replication may be expressed in a arthritic target cell through an inflammatory responsive promoter, wherein the vector optionally encodes IL-10.

The term "operably linked" refers to a linkage of polynucleotide elements in a functional relationship. A nucleic acid sequence is "operably linked" when it is placed into a functional relationship with another nucleic acid sequence. For instance, a promoter or enhancer is operably linked to a coding sequence if it affects the transcription of the coding sequence. Operably linked means that the nucleotide sequences being linked are typically contiguous. However, as enhancers generally function when separated from the promoter by several kilobases and intronic sequences may be of variable lengths, some polynucleotide elements may be operably linked but not directly flanked and may even function in trans from a different allele or chromosome.

The term "repressor of viral replication" refers to a protein, if expressed in a given cell, substantially represses viral replication. As will be appreciated by those of skill in the art, the repressor of viral replication will be dependent on the nature of the parent adenoviral vector from which the recombinant vector of the present invention is derived. For example, in the case of adenoviral vectors or other DNA tumor viruses, the E2F-Rb fusion construct as described in European Patent Application No. 94108445.1 published December 6, 1995 (Publication number. 0 685 493 A1) may be employed. E2F-Rb fusion protein consists of the DNA binding and DP1 heterodimerizations domains of the human E2F transcription factor protein (amino acids 95-286 of wild type E2F) fused to the Rb growth suppression domain (amino acids 379-928 of the wild type Rb protein). The E2F-Rb fusion protein is a potent repressor of E2F-dependent transcription and arrests cells in G1. The DNA binding domain is located at amino acids 128-193 and the dimerization domain is located at 194-289. The sequence of the human E2F-1 protein is available from GenBank under accession number M96577 deposited August 10, 1992. The sequences of E2F from other E2F family members of E2F from other species may be employed when constructing a vector for use in other species. In the situation where the recombinant virus is based on adeno-associated virus (AAV), the rep protein and its derivatives is an effective repressor of viral replication in the absence of adenovirus infection. In the situation where the virus is derived from herpes simplex virus, the ICPO-NX, a deleted form of the immediate early protein ICPO (Liun, *et al.* (1998) J. Virol. 72:7785-7795), protein may be used as an effective repressor of viral replication. Similarly, any protein with dominant negative activity can be used as a repressor of viral replication.

TGF-β and related proteins including activin, inhibins, and BMP are potent natural antiproliferative agents and are believed to play important roles in suppression of tumorigenicity. In its bioactive form, TGF-β is a 25-kDa disulfide-linked homodimer and is expressed in virtually all human tissues. Interestingly, many malignant cell types have retained the expression patterns that are seen in normal cells. TGF-β signals through heteromeric receptor complexes of type I and type II serine/threonine kinase receptors. Among the two receptor kinases, type II receptor possess an intrinsic kinase activity and upon binding to the to TGF-β ligand, the type II receptor heteromerizes with and transphosphorylates type I receptor. Phosphoryaltion of type I receptor activates its kinase activity, which in turn results in the phosphorylation of Smads, which are intracellular effectors. The phosphorylated type I receptor convey signals the inside of the cell resulting in cellular response like growth inhibition. Once inside the nucleus, the Smad complexes are known to activate transcription of target genes either by themselves acting as transcription factors or by regulating the activity of other transcription factors. Transcription factors that are believed to regulated by the TGF-β signal include CTF/NF-1, FAST-1, Sp1, Jun, SRF/TRF, Oct and CREB. The net results of these interactions lead to various known pleiotropic effects of TGF-β.

Transforming growth factor-β (TGF-β) and related proteins are potent inhibitors of proliferation of many cell types. The malignant progression of several tumors of epithelial and hematopoietic origin correlates with the loss of anti-proliferative and anti-invasive action of TGF-β. Failure of the TGF-β signaling has been shown to involve mutations or deletions or lack of expression of the receptors and/or intracellular effectors of the signal transduction pathway. Many malignant tumors that are resistant to TGF-β are also multiply defective for other tumor suppressor genes, thus limiting the utility of tumor suppressor gene replacement for effective therapy.

TGF-β pathway responsive promoters were constructed by incorporating sequences from plasminogen activator inhibitor-1 (PAI-promoter) or binding sites for Smad4/DPC4 (SRE-promoter) upstream of SV40 TATA box. Activities of these promoters were evaluated in transient transfection assays using luciferase as the reporter. The results are presented in Table 2 below:

| **Table 2. Relative Luciferase Activity* (fold)** | | | | | |
|---|---|---|---|---|---|
| **Cell Line** | **TGF-β pathway** | **PAI promoter - TGF- β** | **PAI promoter + TGF-β** | **SRE promoter - TGF-β** | **SRE promoter + TGF-β** |
| Hep3B | Normal | 68.82 | 128.42 | 105.02 | 86.47 |
| 293 | Normal | 51.62 | 88.78 | 9.40 | 46.94 |
| A549 | Normal | 33.26 | 56.76 | 2.87 | 17.93 |
| Panc1 | Normal | 15.36 | 141.80 | 1.14 | 29.03 |
| U87 | Normal | 110.40 | 73.88 | 7.42 | 7.85 |
| MCF-7 | Defective | 18.93 | 10.72 | 1.28 | 1.22 |
| WIDR | Defective | 10.66 | 4.41 | 1.24 | 1.57 |
| MDA-MB468 | Defective | 2.28 | 1.10 | 1.33 | 0.62 |
| AsPC-1 | Defective | 6.56 | 1.24 | 1.79 | 0.76 |
| Caco2 | Defective | 13.01 | 13.8 | 1.18 | 0.96 |
| MicaPaca | Defective | 14.08 | 1.81 | 0.79 | 26.34 |
| *Luciferase activity is expressed relative to the activity obtained with a construct with no promoter. | | | | | |

These results demonstrate that both these promoters are active only in cells with functional TGF-β signal transduction. In a TGF-β pathway defective cell line such as MDA-MB468, which is defective for TGF-β signal transduction because of homozygous deletion of Smad4/DPC4, transfection of PAI-promoter or SRE-promoter operably linked to luciferase and infection with a recombinant adenovirus encoding Smad4 restored the activity of both of the above response elements, further confirming the specificity of these response element-containing promoters for TGF-β pathway as demonstrated by the data presented in Table 3 below.

| **Table 3. Restoration of TGF-β.Responsive Promoter Activity in MDA-MB-468 Cells upon Infection with a Recombinant Adenovirus Encoding Smad4/DPC4 (Relative Luciferase Activity*(fold))** | | | | | |
|---|---|---|---|---|---|
| **Virus** | **Dose** | **PAI promoter -TGF-β** | **PAI promoter + TGF- β** | **SRE promoter - TGF- β** | **SRE promoter + TGF-β** |
| BGCA | 1x10⁸ | 1.0 | 1.53 | 1.00 | 1.00 |
| BGCA | 1x10⁹ | 0.86 | 1.06 | 0.33 | 0.77 |
| DCCA | 1x10⁸ | 1.13 | 4.86 | 8.50 | 139.17 |
| DCCA | 1x10⁹ | 1.93 | 31.60 | 22.00 | 370.84 |
| *Luciferase activity is expressed relative to the activity obtained with a contruct with BGCA in the absence of TGF-β.addition. BGCA is a recombinant adenovirus expressing the β-gal gene. DCCA is a recombinant adenovirus encoding Smad4/DPC4. | | | | | |

A plasmid containing PAI-promoter operably linked to E2F-Rb was then constructed and tested for its ability of selectively repress E2 promoter in cells with intact TGF-β pathway. In transient transfection assays, co-transfection of E2 promoter linked to luciferase with PAI-promoter operably linked to E2F-Rb showed selective repression of E2 promoter activity in 293 cells (with normal TGF-β pathway) and not in MDA-MB 468 cells (with a defective TGF-β pathway) as shown in Table 4, because of selective expression of E2F-Rb in 293 cells. As expected, co-transfection with CMV-E2F-Rb which expresses E2F-Rb in both these cell lines inhibited the activity of E2 promoter in both cell lines.

| **Table 4 Selective Repression of E2 Promoter Activity by PAI-E2F-Rb In 293 Cells v. MDA-MB-468 Cells (Relative Luciferase Activity*(%))** | | |
|---|---|---|
| **Repressor** | **MDA-MB-468 Cells** | **293 Cells** |
| None | 100 | 100 |
| CMV-E2F-Rb | 9.38 | 10.53 |
| PAI-E2F-Rb | 124.02 | 17.38 |
| *Luciferase activity is expressed a a percentage relative to the activity obtained with a construct with no promoter. | | |

p53 pathway responsive promoters were constructed by incorporating known p53 binding sites from either ribosomal gene cluster (RGC-promoter) or high affinity p53 binding sites (p53CON-promoter). Activities of these promoters were evaluated in transient transfection assays using luciferase as the reporter. The results of these experiments are presented in Table 5 below.

| **Table 5. Activities of p53 Responsive Promoters In Various Cell Lines (Relative Luciferase Activity*)** | | | |
|---|---|---|---|
| Cell Line | p53 Status | p53 CON Promoter | RGC-Promoter |
| U87 | Wild-type | 3,921.12 | 112.33 |
| A549 | Wild-type | 647.30 | 16.36 |
| MCF-7 | Wild-type | 445.25 | 12.26 |
| 293 | Wild-type; inactive | 13.03 | 0.62 |
| Hep3B | Null | 4.62 | 1.22 |
| NCI-H358 | Null | 0.35 | 0.56 |
| Panc1 | Mutant | 1.56 | 1.02 |
| WIDR | Mutant | 16.17 | 1.07 |
| MDA-Mb-468 | Mutant | 4.88 | 0.87 |
| AsPC-1 | Mutant | 0.54 | 1.08 |
| Caco-2 | Mutant | 14.59 | 1.13 |
| MicaPaca2 | Unknown | 28.43 | 1.24 |
| *Luciferase activity is expressed relative to the activity obtained with a construct with no promoter. | | | |

Results indicated that both these response elements are active in cells with functional p53. In order to confirm that the p53 responsive promoter activity is due to functional p53, two cell lines WIDR and U87 were cotransfected with RGC promoter driving expression of luciferase gene and either an empty cassette control adenovirus vector (ZZCB) or a recombinant adenovirus constituitively producing p53 under the control of the CMV promoter (FTCB). The results of these experiments are presented in Table 6.

| **Table 6. Restoration/enhancement of p53 Responsive Promoter Activity in WIDR and U87 cells Upon Infection with a Recombinant Adenovirus Encoding p53 (Relative Luciferase Activity*)** | | | |
|---|---|---|---|
| **Virus** | **Dose** | **WIDR** | **U87** |
| ZZCB | 1 x 10⁸ | 1.0 | 1.0 |
| ZZCB | 1 x 10⁹ | 1.25 | 0.67 |
| FTCB | 1 x 10⁸ | 4.71 | 20.30 |
| FTCB | 1 x 10⁹ | 29.00 | 109.95 |
| *Luciferase activity is expressed relative to the activity obtained with ZZCB infection. | | | |

As can be seen from the data presented, activity of p53-responsive promoter increases in a dose-dependent manner with increasing p53 activity.

Recombinant adenovirus vectors encoding E2F-Rb fusion coding sequence under the control of either TGF-β pathway-responsive promoter (PAI or SRE) or p53 pathway-responsive promoter (RGC or p53CON) were generated. In addition, the gene encoding green fluorescent protein was also incorporated in the vectors as a reporter. The resulting viruses were tested for their ability to selectively replicate and kill cells with either TGF-β or p53 pathway defects in cytopathic effect (CPE) assays. The results are shown in Figure 1 of the attached drawings. In MRC9 (p53 pathway positive and TGF-β pathway positive) the wild type adenovirus was able to replicate and induce CPE even at low concentrations (1x 10⁶ particles/ml), whereas the TGF-β or p53 pathway targeted vectors failed to induce CPE at that concentration. Only at the highest dose tested (1x 10⁸ particles/ml), pathway-targeted vectors showed some CPE. In contrast in cell line such as WIDR (defective in both p53 and TGF-β pathways) and Hep3B (p53 null and TGF-β pathway defective in the absence of exogenous TGF-β) pathway-targeted vectors were effective in inducing CPE similar to the wild type virus even at low concentrations (1 x 10⁶ particles/ml). Fluorescence microscopy of Hep3B cells infected with pathway targeted vectors (SRE-Ad and p53Con-Ad) revealed high-level expression of the transgene and the spread of the virus within the culture, even when infected at a low particle concentration (1x 10⁵ particles/ml exposed for two hours). In contrast, Hep3B cells infected with the replication-defective (E1A-deleted) GFP encoding adenovirus (GFCB) at the same concentration (1x10⁵ particles/ml), showed no GFP expression.

As previously indicated, the vectors of the present invention are capable of selective replication and lysis of the target cell under selective conditions. However, this is not meant to imply that additional layers of selectivity or toxicity cannot be engineered into these vectors. The present invention also provides recombinant adenoviruses containing additional modifications to the viral genome such as targeting modifications, transgene expression cassettes or modifications to the viral genome to facilitate selective replication in a given cell type or phenotypic state.

The term "targeting modification" refers to modifications to the viral genome designed to result in preferential infectivity of a particular cell type. Cell type specificity or cell type targeting may also be achieved in vectors derived from viruses having characteristically broad infectivities such as adenovirus by the modification of the viral envelope proteins. For example, cell targeting has been achieved with adenovirus vectors by selective modification of the viral genome knob and fiber coding sequences to achieve expression of modified knob and fiber domains having specific interaction with unique cell surface receptors. Examples of such modifications are described in Wickham, *et al.* (1997) J. Virol 71(11):8221-8229 (incorporation of RGD peptides into adenoviral fiber proteins); Arnberg, *et al.* (1997) Virology 227:239-244 (modification of adenoviral fiber genes to achieve tropism to the eye and genital tract); Harris and Lemoine (1996) TIG 12(10):400-405; Stevenson, *et al.* (1997) J. Virol. 71(6):4782-4790; Michael, *et al*.(1995) gene therapy 2:660-668 (incorporation of gastrin releasing peptide fragment into adenovirus fiber protein); and Ohno, *et al.* (1997) Nature Biotechnology 15:763-767 (incorporation of Protein A-IgG binding domain into Sindbis virus). Other methods of cell specific targeting have been achieved by the conjugation of antibodies or antibody fragments to the envelope proteins (see, e.g. Michael, *et al.* (1993) J. Biol. Chem. 268:6866-6869, Watkins, *et al.* (1997) Gene Therapy 4:1004-1012; Douglas, *et al.* (1996) Nature Biotechnology 14: 1574-1578. Alternatively, particular moieties may be conjugated to the viral surface to achieve targeting (See, e.g. Nilson, *et al.* (1996) Gene Therapy 3:280-286 (conjugation of EGF to retroviral proteins). These recombinantly modified vectors may be produced in accordance with the practice of the present invention.

The term "transgene expression cassette" refers to a promoter functional in the target cell operably linked to a therapeutic transgene. The term promoter refers to nucleotide sequences which affect the transcription of another nucleotide sequence. Examples of promoters include weak constitutive promoters, temporal viral promoters or regulatable promoters.

The term "temporal promoters" refers to promoters which drive transcription or the therapeutic transgene at a point later in the viral cycle relative to the promoter controlling expression of the pathway-responsive promoter. Examples of such temporally regulated promoters include the adenovirus major late promoter (MLP), other promoters such as E3. In the preferred practice of the invention, the MLP promoter is employed. For herpes simplex viruses, the Latent Activated Promoters could be used.

The term "regulatable promoters" refers to inducible promoters, tissue specific or tumor specific promoters. The term "inducible promoter" refers to promoters which facilitate transcription of the therapeutic transgene preferable (or solely) under certain conditions and/or in response to external chemical or other stimuli. Examples of inducible promoters are known in the scientific literature (See, e.g. Yoshida and Hamada (1997) Biochem. Biophys. Res. Comm. 230:426-430; Iida, *et al.* (1996) J. Virol. 70(9):6054-6059; Hwang, *et al.* (1997) J. Virol 71(9):7128-7131; Lee*, et al.* (1997) Mol. Cell. Biol. 17(9):5097-5105; and Dreher, *et al.* (1997) J. Biol. Chem. 272(46); 29364-29371. Examples of radiation inducible promoters are described in Manome, *et al.* (1998) Human Gene Therapy 9:1409-1417). An additional level of selectivity can be imparted through the use of tissue specific of tumor specific promoters. Tissue specific and tumor specific promoters are well known in the art and include promoters active preferentially in smooth muscle (α-actin promoter), pancreas specific (Palmiter, *et al.* (1987) Cell 50:435), liver specific Rovet, et.al. (1992) J. Biol. Chem.. 267:20765; Lemaigne, *et al.* (1993) J. Biol. Chem.. 268:19896; Nitsch, *et al.* (1993) Mol. Cell. Biol. 13:4494), stomach specific (Kovarik, *et al.* (1993) J. Biol. Chem.. 268:9917, pituitary specific (Rhodes, *et al.* (1993) Genes Dev. 7:913, prostate specific, etc.

The term "therapeutic transgene" refers to a nucleotide sequence the expression of which in the target cell produces a therapeutic effect. The term therapeutic transgene includes but is not limited to tumor suppressor genes, antigenic genes, cytotoxic genes, cytostatic genes, pro-drug activating genes, apoptotic genes, pharmaceutical genes or anti-angiogenic genes. The vectors of the present invention may be used to produce one or more therapeutic transgenes, either in tandem through the use of IRES elements or through independently regulated promoters.

The term "tumor suppressor gene" refers to a nucleotide sequence, the expression of which in the target cell is capable of supressing the neoplastic phenotype and/or inducing apoptosis. Examples of tumor suppressor genes useful in the practice of the present invention include the p53 gene, the APC gene, the DPC-4/Smad4 gene, the BRCA-1 gene, the BRCA-2 gene, the WT-1 gene, the retinoblastoma gene (Lee, *et al.* (1987) Nature 329:642), the MMAC-1 gene, the adenomatous polyposis coli protein (Albertsen, *et al.,* United States Patent 5,783,666 issued July 21, 1998), the deleted in colon carcinoma (DCC) gene, the MMSC-2 gene, the NF-1 gene, nasopharyngeal carcinoma tumor suppressor gene that maps at chromosome 3p21.3. (Cheng, *et al.* 1998. Proc. Nat. Acad. Sci. 95:3042-3047), the MTS 1 gene, the CDK4 gene, the NF-1 gene, the NF2 gene, and the VHL gene.

The term "antigenic genes" refers to a nucleotide sequence, the expression of which in the target cells results in the production of a cell surface antigenic protein capable of recognition by the immune system. Examples of antigenic genes include carcinoembryonic antigen (CEA), p53 (as described in Levine, A. PCT International Publication No. WO94/02167 published February 3, 1994). In order to facilatate immune recognition, the antigenic gene may be fused to the MHC class I antigen.

The term "cytotoxic gene" refers to nucleotide sequence, the expression of which in a cell produces a toxic effect. Examples of such cytotoxic genes include nucleotide sequences encoding pseudomonas exotoxin, ricin toxin, diptheria toxin, and the like.

The term "cytostatic gene" refers to nucleotide sequence, the expression of which in a cell produces an arrest in the cell cycle. Examples of such cytostatic genes include p21, the retinoblastoma gene, the E2F-Rb fusion protein gene, genes encoding cyclin dependent kinase inhibitors such as P16, p15, p18 and p19, the growth arrest specific homeobox (GAX) gene as described in Branellec*, et al.* (PCT Publication WO97/16459 published May 9, 1997 and PCT Publication WO96/30385 published October 3, 1996) .

The term "cytokine gene" refers to a nucleotide sequence, the expression of which in a cell produces a cytokine. Examples of such cytokines include GM-CSF, the interleukins, especially IL-1,IL-2, IL-4, IL-12, IL-10, IL-19, IL-20, interferons of the α, β and γ subtypes especially interferon α-2b and fusions such as interferon α-2α-1.

The term "chemokine gene" refers to a nucleotide sequence, the expression of which in a cell produces a cytokine. The term chemokine refers to a group of structurally related low-molecular cytokines weight factors secreted by cells are structurally related having mitogenic, chemotactic or inflammatory activities. They are primarily cationic proteins of 70 to 100 amino acid residues that share four conserved cysteine residues. These proteins can be sorted into two groups based on the spacing of the two amino-terminal cysteines. In the first group, the two cysteines are separated by a single residue (C-x-C), while in the second group, they are adjacent (C-C). Examples of member of the 'C-x-C' chemokines include but are not limited to platelet factor 4 (PF4), platelet basic protein (PBP), interleukin-8 (IL-8), melanoma growth stimulatory activity protein (MGSA), macrophage inflammatory protein 2 (MIP-2), mouse Mig (m119), chicken 9E3 (or pCEF-4), pig alveolar macrophage chemotactic factors I and I (AMCF-I and -II), pre-B cell growth stimulating factor (PBSF),and IP10. Examples of members of the 'C-C' group include but are not limited to monocyte chemotactic protein 1 (MCP-1), monocyte chemotactic protein 2 (MCP-2), monocyte chemotactic protein 3 (MCP-3), monocyte chemotactic protein 4 (MCP-4), macrophage inflammatory protein 1 α (MIP-1-α), macrophage inflammatory protein 1 β (MIP-1-β), macrophage inflammatory protein 1 γ (MIP-1-γ), macrophage inflammatory protein 3-α (MIP-3-α, macrophage inflammatory protein 3 β (MIP-3-β), chemokine (ELC), macrophage inflammatory protein 4 (MIP-4), macrophage inflammatory protein 5 (MIP-5), LD78 β, RANTES, SIS-epsilon (p500), thymus and activation-regulated chemokine (TARC), eotaxin, I-309, human protein HCC-1/NCC-2, human protein HCC-3, mouse protein C10.

The term "pharmaceutical protein gene" refers to nucleotide sequence, the expression of which results in the production of protein have pharmaceutically effect in the target cell. Examples of such pharmaceutical genes include the proinsulin gene and analogs (as described in PCT International Patent Application No. WO98/31397, growth hormone gene, dopamine, serotonin, epidermal growth factor, GABA, ACTH, NGF, VEGF (to increase blood perfusion to target tissue, induce angiogenesis, PCT publication WO98/32859 published July 30, 1998), thrombospondin etc.

The term "pro-apoptotic gene" refers to a nucleotide sequence, the expression thereof results in the programmed cell death of the cell. Examples of pro-apoptotic genes include p53, adenovirus E3-11.6K(derived from Ad2) or adenovirus E3-10.5K (derived from Ad), the adenovirus E4orf4 gene, p53 pathway genes, and genes encoding the caspases.

The term "pro-drug activating genes" refers to nucleotide sequences, the expression of which, results in the production of protein capable of converting a non-therapeutic compound into a therapeutic compound, which renders the cell susceptible to killing by external factors or causes a toxic condition in the cell. An example of a prodrug activating gene is the cytosine deaminase gene. Cytosine deaminase converts 5-fluorocytosine (5-FC) to 5-fluorouracil (5-FU), a potent antitumor agent. The lysis of the tumor cell provides a localized burst of cytosine deaminase capable of converting 5FC to 5FU at the localized point of the tumor resulting in the killing of many surrounding tumor cells. This results in the killing of a large number of tumor cells without the necessity of infecting these cells with an adenovirus (the so-called bystander effect"). Additionally, the thymidine kinase (TK) gene (see e.g. Woo, *et al.* United States Patent No. 5,631,236 issued May 20, 1997 and Freeman, *et al.* United States Patent No. 5,601,818 issued February 11, 1997) in which the cells expressing the TK gene product are susceptible to selective killing by the administration of gancyclovir may be employed.

The term "anti-angiogenic" genes refers to a nucleotide sequence, the expression of which results in the extracellular secretion of anti-angiogenic factors. Anti-angiogenesis factors include angiostatin, inhibitors of vascular endothelial growth factor (VEGF) such as Tie 2 (as described in PNAS(USA)(1998) 95:8795-8800), endostatin.

It will be readily apparent to those of skill in the art that modifications and or deletions to the above referenced genes so as to encode functional subfragments of the wild type protein may be readily adapted for use in the practice of the present invention. For example, the reference to the p53 gene includes not only the wild type protein but also modified p53 proteins. Examples of such modified p53 proteins include modifications to p53 to increase nuclear retention, deletions such as the Δ13-19 amino acids to eliminate the calpain consensus cleavage site, modifications to the oligomerization domains (as described in Bracco, *et al.* PCT published application WO97/0492 or United States Patent No. 5,573,925).

It will be readily apparent to those of skill in the art that the above therapeutic genes may be secreted into the media or localized to particular intracellular locations by inclusion of a targeting moiety such as a signal peptide or nuclear localization signal (NLS). Also included in the definition of therapeutic transgene are fusion proteins of the therapeutic transgene with the herpes simplex virus type 1 (HSV-1) structural protein, VP22. Fusion proteins containing the VP22 signal, when synthesized in an infected cell, are exported out of the infected cell and efficiently enter surrounding non-infected cells to a diameter of approximately 16 cells wide. This system is particularly useful in conjunction with transciptionally active proteins (e.g. p53) as the fusion proteins are efficiently transported to the nuclei of the surrounding cells. See, e.g.Elliott, G. & O'Hare, P. Cell. 88:223-233:1997; Marshall, A. & Castellino, A. Research News Briefs. Nature Biotechnology. 15:205:1997; O'Hare, *et al.* PCT publication WO97/05265 published February 13, 1997. A similar targeting moiety derived from the HIV Tat protein is also described in Vives, *et al.* (1997) J. Biol. Chem. 272:16010-16017.

Additionally modifications to increase the potency of the vectors of the present invention include but are not limited to alterations within E1, introductions of mutations in E4 to increase the cytotoxicity (Muller, et al (1992) J. Virol. 66:5867-5878), up-regulation of viral death proteins such as E4orf4 or E3 11.6K proteins.

In the preferred practice of the invention as exemplified herein, the vector of the present invention comprises a conditionally replicating adenovirus containing a p53 or TGF-β pathway-responsive promoter driving expression of the E2F-Rb fusion protein, additionally comprising the cytosine deaminase gene is under control the temporal E3 promoter. In such instances the cytosine deaminase is produced only in tumor cells following viral replication. In the preferred practice of the invention, the prodrug converting gene is under the control of a relatively weak or tissue-specific or tumor specific promoter.

In another embodiment of the invention, the vector comprises a recombinant adenvirus with a p53 or TGF-β pathway-responsive promoter driving expression of the E2F-Rb fusion protein and a transgene expression cassette expressing cytosine deaminase alone or in a bi-cistronic express cassette containing cytosine deaminase, and IRES element and the MIP-3-alpha protein. Because cytosine deaminase is expressed, tumor cell killing is achieved upon administration of a prodrug such as 5-fluorocytosine. Low level expression of MIP-3-alpha will aid in the development of antitumor immunity.

Multi-level, multi-functional or multiple pathway responsive cascade are used interchangeably herein to refer to the construction of a pathway responsive elements so as to produce a therapeutic effect where more than one pathway can be probed simultaenously. It will be apparent to those of skill in the art that the vector control elements as described above may be combined to provide multiple layers of selectivity to the vectors of the present invention. As an example of multilevel control, it would be possible to design a conditionally replicating adenovirus that can replicate and kill cells that have defects in either Rb, TGF-β or p53 pathway. Such a vector would include expression of a dominant negative inhibitor of viral replication controlled by a p53-responsive promoter as a primary level of control. As a result, in any cell (such as all normal cells) with functional p53, but not in cell with inactive p53, the dominant negative inhibitor is expressed and the viral replication is blocked. However, this vector may replicate in tumor cells with functional p53 but with defects in other pathways such as TGF-β and Rb pathway. Therefore, additional levels of control can be inserted which would essentially inactivate functional p53 in tumor cells, thus allowing viral replication, when other pathways are defective.

As a second level of control, the same vector would also consist of an expression cassette containing adenovirus E1B-55K protein, which can functionally inactivate p53, under the control of a promoter that is active only in TGF-β pathway defective cells. A promoter that is active only in TGF-β pathway defective cells can be constructed by inserting repressor binding sites within a promoter such that expression ot the repressor using a TGF-β responsive promoter elsewhere on the vector will lead to blockage of the promoter activity in cells with intact TGF-β signaling. On the other hand, in cells where the TGF-β pathway is defective, the repressor is not synthesized, thus the promoter will be active. Because of the expression of E1B-55K from the promoter that is active only in cells in which TGF-β pathway is defective, endogenous p53 is inactivated. Alternatively, any natural promoter that is down-regulated because of TGF-β signaling can be used. The inclusion of the above two expression cassettes will ensure that the dominant negative inhibitor is expressed only when both p53 and TGF-β pathways are normal (leads to blockage of replication), but not when one or both of them are defective (leads to viral replication).

As a third level of control, expression of a protein such as Smad7 or any other dominant negative form of Smad, which is capable of inactivating TGF-β pathway (Nakao *et al.,* Nature 1997 Oct 9;389(6651):631-635) controlled by a E2F-responsive promoter is achieved in the same vector. A E2F-responsive promoter (such as E2F1 promoter, E2 promoter or a synthetic promoter with multiple E2F-binding sites upstream of a minimal promoter such as SV40 TATA box) is active when Rb pathway is defective. Because of this third level of control, in cells with defective Rb pathway, Smad7 is expressed which in turn inactivates Smad4 and blocks TGF-β signal transduction. Therefore, E1B-55K is made and p53 is inactivated leading to lack of expression of the dominant negative inhibitor. Therefore, viral replication can proceed unhindered. On the other hand, if Rb pathway is normal, Smad7 is not synthesized, thus TGF-β signal transduction proceeds normally. Therefore, E1B-55K is not made, resulting in a functional p53 capable of expressing the dominant negative inhibitor to block viral replication.

With a above three levels of control, a defect in any one or two or all of the three pathways (Rb, TGF-β and p53) would ultimately lead to viral replication and cell lysis. Viral replication will not occur only when all three pathways are functional as in normal cells.

As can be appreciated by those of skill in the art, the vectors of the present invention can be used in many variations for the detection and treatment of a wide variety of pathway defects using a variety of pathway-responsive promoters. However, in the preferred practice of the invention as exemplified herein, the recombinant adenoviral vector is derived from genus adenoviridiae. Particularly preferred viruses are derived from the human adenovirus type 2 or type 5. When an adenoviral vector is used as the parent vector, the preferred inhibitors of viral replication is the E2F-RB fusion protein.

In the preferred practice of the invention when using the vectors of the present invention to treat diseases associated with uncontrolled cellular proliferation, it is preferred that an adenoviral vector be employed containing specific deletions in the E1A region so as to eliminate the ability of the E1a gene product to bind to the p300 and Rb proteins while retaining the transactivating function of the E1a CR3 domain. The vectors of the present contain deletions in the E1a coding sequence to eliminate p300 and p105-Rb binding sites in the 13S coding sequence. In the preferred practice of the invention, the p300 binding deletions are represented by deletions of amino acids from about 4 to about 25 or from about 36 to about 49.

The deletions in the E1a 289R coding sequence necessary to achieve elimination of p300 and pRb binding are preferably as minimal as possible to prevent major disruption of the secondary and tertiary structure of the E1a 289R protein. In order to eliminate p300 binding it is preferred that a mutation be introduced in the DNA sequence encoding the p300 binding domains of 289R. Deletions of less than about 30 amino acids in the C-terminal region to eliminate p300 binding are preferred, although smaller modifications are preferred. For example, a deletion of amino acids 4 to 25 (dl1101), from about amino acid 30 to amino acid 49 (dl1103) and more particularly 36 to 49 are alternatively preferred to eliminate p300 binding. Point mutations sufficient to disrupt binding p300 are particularly preferred. For example, a point mutation of the second amino acid from arginine to glycine (Arg2 → Gly2) in the 289R protein has been demonstrated to disrupt p300 binding (See e.g., pm563, Whyte, et al, (1989) Cell 56:67-75). Similarly, in regard to eliminating pRb105 binding, minimal modifications are preferred. Elimination of selective amino acids in the pRb105 binding domain such as amino acid 111-123 (dl1107) and amino acids 124-127 (dl1108) are preferred. Deletion of amino acids 111-123 (dl1107) is particularly preferred in that it retains the p107 binding activity of the 289R protein.

Additionally, the elimination of amino acids from approximately 219 to 289 of the E 1 a 289R protein and 173 to 243 of the E1A 243R protein may be introduced. For example, by introducing a point mutation at position corresponding to position 1131 of the human Ad5 genome (i.e., changing the cytosine¹¹³¹ to a guanine) creates a stop codon. This mutation results in the elimination of amino acids 219 to 289 of the E1a 289R protein and 173 to 243 of the E1A 243R protein. This mutation is made optionally in addition to the deletions in Rb and p300 binding described above. Additional examples of such parent vectors are described in co-pending United States Patent Application Serial Numbers 60/104,317 and 08/09/172,684 filed October 15, 1998.

In the preferred practice of the invention the therapeutic gene is a pro-drug activating gene e.g., cytosine deaminase. In the preferred practice of the invention to induce anti-tumor immunity, the vector of the present invention encodes MIP-3-alpha.

Preferred promoters for the expression of the therapeutic gene are the temporal promoters such as MLP and E3.

In adenoviral constructs, the elimination or delay in action of the Ad E3-11.6 K protein to minimize cell lysis induced by adenovirus until replication is achieved. In particular, the elimination of the native E3-11.6K/10.5K gene would be preferable. This would minimize the immune response until after the initial round of localized spreading occurs. At this later time, once apoptosis of the initially infected cells is achieved and localized virus spreading is permitted, the immune response would be advantageous. The 11.6K protein (or the corresponding E3-10.5K protein of Ad5) is a pro-apoptotic gene and may be used to achieve cell killing in tumor cells. However, as one wishes to delay the early lysis of the target cell to maximize viral replication, it is preferred that the 11.6K/10.5K gene be placed under control of a temporal promoter such as the MLP promoter to delay onset of its apoptotic effect.

### Pharmaceutical Formulations

The present invention further provides a pharmaceutically acceptable formulation of the recombinant viruses in combination with a carrier. The vectors of the present invention may be practice formulated for dose administration in accordance with conventional pharmaceutical with the addition of carriers and excipients. Dosage formulations may include intravenous, intratumoral, intramuscular, intraperitoneal, topical, matrix or aerosol delivery.

The term "carrier" refers to compounds commonly used on the formulation of pharmaceutical compounds used to enhance stability, sterility and deliverability of the therapeutic compound. When the virus is formulated as a solution or suspension, the delivery system is in an acceptable carrier, preferably an aqueous carrier. A variety of aqueous carriers may be used, e.g., water, buffered water, 0.8% saline, 0.3% glycine, hyaluronic acid and the like. These compositions may be sterilized by conventional, well known sterilization techniques, or may be sterile filtered. The resulting aqueous solutions may be packaged for use as is, or lyophilized, the lyophilized preparation being combined with a sterile solution prior to administration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorption monolaurate, triethanolamine oleate, etc.

The present invention further provides pharmaceutical formulations of the viruses of the present invention with a carrier and a delivery enhancing agent(s). The terms delivery enhancers" or "delivery enhancing agents" are used interchangeably herein and includes one or more agents which facilitate uptake of the virus into the target cell. Examples of delivery enhancers are described in co-pending United States Patent Applications Serial No. 09/112,074 filed July 8, 1998 and 08/938,089 filed September 26,1997. Examples of such delivery enhancing agents include detergents, alcohols, glycols, surfactants, bile salts, heparin antagonists, cyclooxygenase inhibitors, hypertonic salt solutions, and acetates. Alcohols include for example the aliphatic alcohols such as ethanol, N-propanol, isopropanol, butyl alcohol, acetyl alcohol. Glycols include glycerine, propyleneglycol, polyethyleneglycol and other low molecular weight glycols such as glycerol and thioglycerol. Acetates such as acetic acid, gluconic acid, and sodium acetate are further examples of delivery-enhancing agents. Hypertonic salt solutions like 1M NaCl are also examples of delivery-enhancing agents. Examples of surfactants are sodium dodecyl sulfate (SDS) and lysolecithin, polysorbate 80, nonylphenoxypolyoxyethylene, lysophosphatidylcholine, polyethyleneglycol 400, polysorbate 80, polyoxyethylene ethers, polyglycol ether surfactants and DMSO. Bile salts such as taurocholate, sodium tauro-deoxycholate, deoxycholate, chenodesoxycholate, glycocholic acid, glycochenodeoxycholic acid and other astringents such as silver nitrate may be used. Heparin-antagonists like quaternary amines such as protamine sulfate may also be used. Cyclooxygenase inhibitors such as sodium salicylate, salicylic acid, and ron-steroidal antiinflammatory drug (NSAIDS) like indomethacin, naproxen, diclofenac may be used.

The term "detergent" includes anionic, cationic, zwitterionic, and nonionic detergents. Exemplary detergents include but are not limited to taurocholate, deoxycholate, taurodeoxycholate, cetylpyridium, benalkonium chloride, Zwittergent 3-14 detergent, CHAPS (3-[(3-Cholamidopropyl) dimethylammoniol]-1-propanesulfonate hydrate), Big CHAP, Deoxy Big CHAP, Triton-X-100 detergent, C12E8, Octyl-B-D-Glucopyranoside, PLURONIC- F68 detergent, Tween 20 detergent, and TWEEN 80 detergent (CalBiochem Biochemicals).

Unit dosage formulations of the present invention may be included in a kit of products containing the recombinant virus of claim 1 in lyophilized form and a solution for reconstitution of the lyophilized product. Recombinant viruses of the present invention may be lyophilized by conventional procedures and reconstituted.

The vectors of the present invention may also be administered in combination with calpain inhibitors. The "calpain inhibitor" (abbreviated "CI") refers to a compound which inhibits the proteolytic action of calpain-I, e.g. µ-calpains. The term calpain inhibitors as used herein includes those compounds having calpain I inhibitory activity in addition to or independent of their other biological activities. A wide variety of compounds have been demonstrated to have activity in inhibiting the proteolytic action of calpains. Examples of calpain inhibitors are useful in the practice of the present invention include N-acetyl-leu-leu-norleucinal also known as "calpain inhibitor 1." Calpain inhibitors have been observed to increase infectivity of cells with respect to viral vectors, to enhance transcription from promoters by increasing levels of NF-kappaB and AP-1 transcription factors and to diminish the CTL response to adenoviral vectors. Consequently, the formulations and methods of the present invention may optionally include calpain inhibitors. Calpain inhibitors and their applications are described in Atencio, *et al.* co-pending United States Patent Application Serial No. 60/104,321 and 09/073,076 filed October 15, 1998.

### Methods of Use

The present invention provides a method of killing a cell with a regualtory pathway defect by contacting the target cell with a selectively replicating vector of the present invention. In one embodiment of the invention as exemplified herein, the cell containing a pathway defect is a neoplastic cell. The term "neoplastic cell" is a cell displaying an aberrant growth phenotype characterized by independence of normal cellular growth controls. As neoplastic cells are not necessarily replicating at any given time point, the term neoplastic cells comprise cells which may be actively replicating or in a temporary non-replicative resting state (G 1 or G0). Localized populations of neoplastic cells are referred to as neoplasms. Neoplasms may be malignant or benign. Malignant neoplasms are also referred to as cancers. The term cancer is used interchangeably herein with the term tumor. Neoplastic transformation refers the conversion of a normal cell into a neoplastic cell, often a tumor cell.

The present invention provides a method of ablating neoplastic cells in a mammalian organism *in vivo* by the administration of a pharmaceutically acceptable formulation of the recombinant adenovirus of the present invention. The term "ablating" means the substantial reduction of the population of viable neoplastic cells so as to alleviate the physiological maladictions of the presence of the neoplastic cells. The term "substantial" means a reduction in the population of viable neoplastic cells in the mammalian organism by greater than approximately 20% of the pretreatment population. The term "viable" means having the uncontrolled growth and cell cycle regulatory characteristics of a neoplastic cell. The term "viable neoplastic cell" is used hereing to distinguish said cells from neoplastic cells which are no longer capable of replication. For example, a tumor mass may remain following treatment, however the population of cells comprising the tumor mass may be dead. These dead cells have been ablated and lack the ability to replicate, even though some tumor mass may remain.

The term "mammalian organism" includes, but is not limited to, humans, pigs, horses, cattle, dogs, cats. Preferably one employs an adenoviral vector endogenous to the mammalian type being treated. Although it is generally favored to employ a virus from the species to be treated, in some instances it may be advantageous to use vectors derived from different species which possess favorable pathogenic features. For example, it is reported (WO 97/06826 published April 10, 1997) that ovine adenoviral vectors may be used in human gene therapy to minimize the immune response characteristic of human adenoviral vectors. By minimizing the immune response, rapid systemic clearance of the vector is avoided resulting in a greater duration of action of the vector.

The vectors of the present invention are particularly applicable to the treatment of tumors associated with a lack of TGF-β antiproliferative action inluding but not limited to breast carcinomas, hepatomas, gastric, colon and skin tumors, as well as B and T lymphomas (Markowitz and Roberts, 1996). Mutations of type II TGF-β receptor have been identified in colon, gastric, head and neck squamous carcinomas. Mutations or deletions of type I receptors have also been found in Kaposi's sarcoma, breast, ovarian and colorectal tumors. Among the intracellular effectors of TGF-β signaling, Smads, Smad4/DPC4 was identified as a candidate tumor suppressor genes that was altered in nearly 50% of the pancreatic cancers. Alteration of DPC4 gene has also been found in colon, breast and ovarian tumors although at much lower frequency. The vectors of the present invention are particularly applicable to the treatment of TGF-β insensitive tumors such as pancreatic cancer.

The vectors of the present invention are also applicable to the treatment of tumor cells containing p53-pathway defects. These tumors include those possessing alterations in p53, mdm2 and p14/p19ARF(INK4a gene). The vectors of the present invention are also useful in treating cancer cells with Rb pathway defects. Rb pathway defects result from alterations in Rb, cyclin D, cyclin dependent kinase (such as CDK4) and p16 (INK4a gene).

While the present invention provides a method of use of the recombinant adenoviruses alone, the recombinant adenoviruses of the present invention and formulations thereof may be employed in combination with conventional chemotherapeutic agents or treatment regimens. Examples of such chemotherapeutic agents include inhibitors of purine synthesis (e.g., pentostatin, 6-mercaptopurine, 6thioguanine, methotrexate) or pyrimidine synthesis (e.g. Pala, azarbine), the conversion of ribonucleotides to deoxyribonucleotides (e.g. hydroxyurea), inhibitors of dTMP synthesis (5-fluorouracil), DNA damaging agents (e.g. radiation, bleomycines, etoposide, teniposide, dactinomycine, daunorubicin, doxorubicin, mitoxantrone, alkylating agents, mitomycin, cisplatin, procarbazine) as well as inhibitors of microtubule function (e.g vinca alkaloids and colchicine). Chemotherapeutic treatment regimens refers primarily to non-chemical procedures designed to ablate neoplastic cells such as radiation therapy. Examples of combination therapy when the therapeutic gene is p53 are described in Nielsen, *et al.* WO/9835554A2 published August 20,1998.

The immunological response is significant to repeated *in vivo* administration of viral vectors. Consequently, the vectors of the present invention may be administered in combination with immunosuppressive agents. Examples of immunosuppressive agents include cyclosporine, azathioprine, methotrexate, cyclophosphamide, lymphocyte immune globulin, antibodies against the CD3 complex, adrenocorticosteroids, sulfasalzaine, FK-506, methoxsalen, and thalidomide.

The present invention also provides a method of ablating neoplastic cells in a population of normal cells contaminated by said neoplastic cells *ex vivo* by the administration of a recombinant adenovirus of the present invention to said population. An example of the application of such a method is currently employed in *ex vivo* applications such as the purging of autologous stem cell products commonly known as bone marrow purging. The term "stem cell product" refers to a population of hematopoietic, progenitor and stem cells capable of reconstituting the long term hematopoietic function of a patient who has received myoablative therapy. Stem cell products are conventionally obtained by apheresis or mobilized or non-mobilized peripheral blood. Apheresis is conventionally achieved through the use of known procedures using commercially available apheresis apparatus such as the COBE Spectra Apheresis System, commercially available from COBE International, 1185 Oak Street, Lakewood, CO. It is preferred that treatment conditions be optimized to achieve a "3-log purge" (i.e. removal of approximately 99.9% of the tumor cells from the stem cell product) and most preferably a "5-log purge" (removal of approximately 99.999% of tumor cells from the stem cell product). In the preferred practice of the invention, a stem cell product of 100 ml volume would be treated at a particle number to nucleated cell ratio of approximately 2 x 10¹¹ of the vectors of the present invention for a period of approximately 4 hours at 37C.

### Diagnostic Applications

In addition to therapeutic applications described above, the vectors of the present invention are also useful for diagnostic purposes. For example, the vectors of the present invention may incorporate a reporter gene which is expressed upon viral infection or replication. The term "reporter gene" refers to a gene whose product is capable of producing a detectable signal alone or in combination with additional elements. Examples of reporter genes includes the β-galactosidase gene, the luciferase gene, the green fluorescent protein gene, nucleotide sequences encoding proteins detectable by imaging systems such as X-rays or magnetic field imaging systems (MRI). Such vectors would be useful to detect the presence of a functional pathway (e.g. p53 or TGF-beta pathway. Alternatively, such vectors may also be employed to express a cell surface protein capable of recognition by a binding molecule such as a fluorescently labelled antibody. Alternatively where the pathway-responsive promoter is used to drive a repressor of viral replication (e.g E2F-Rb) late viral promoters (for example E2 which is turned off by E2F-Rb or any other promoter with repressor binding sites for example E2F binding sites) could be used to drive the reporter gene for diagnostic applications where the pathway-responsive promoter is off. These diagnostic constructs may be used for diagnostic purposes *in vivo* or *in vitro.* Examples of *in vivo* applications include imaging applications such as X-ray, CT scans or Magnetic Resonance Imaging (MRI).

### Methods of Preparing The Vectors

The present invention further provides a method of producing the recombinant viruses described above, said method comprising the steps of: :
a. infecting a producer cell with a recombinant virus
b. culturing said infected producer cell under conditions so as to permit replication of the viral genome in the producer cell,
c. harvesting the producer cells, and
d. purifying the recombinant virus.

The term "infecting" means exposing the recombinant virus to the producer cell under conditions so as to facilitate the infection of the producer cell with the recombinant adenovirus. In cells which have been infected by multiple copies of a given virus, the activities necessary for viral replication and virion packaging are cooperative. Thus, it is preferred that conditions be adjusted such that there is a significant probability that the producer cells are multiply infected with the virus. An example of a condition which enhances the production of virus in the producer cell is an increased virus concentration in the infection phase. However, it is possible that the total number of viral infections per producer cell can be overdone, resulting in toxic effects to the cell. Consequently, one should strive to maintain the infections in the virus concentration in the range of 1x 10⁶ to 1x 10¹⁰, preferably about 1x 10⁹, virions per ml. Chemical agents may also be employed to increase the infectivity of the producer cell line. For example, the present invention provides a method to increase the infectivity of producer cell lines for viral infectivity by the inclusion of a calpain inhibitor. Examples of calpain inhibitors useful in the practice of the present invention include calpain inhibitor 1 (also known as N-acetyl-leucyl-leucyl-norleucinal, commercially available from Boehringer Mannheim). Calpain inhibitor 1 has been observed to increase the infectivity of producer cell lines to recombinant adenovirus.

The term "producer cell" means a cell capable of facilitating the replication of the viral genome of the recombinant adenovirus to be produced. A variety of mammalian cell lines are publicly available for the culture of recombinant adenoviruses. For example, the 293 cell line (Graham and Smiley (1977) J. Gen. Virol. 36:59-72) has been engineered to complement the deficiencies in E1 function and is a preferred cell line for the production of the current vectors. Examples of other producer cells include HeLa cells, PERC.6 cells (as described in publication WO/97/00326, application serial No. PCT/NL96/00244.

The term "culturing under conditions to permit replication of the viral genome" means maintaining the conditions for the infected producer cell so as to permit the virus to propagate in the producer cell. It is desirable to control conditions so as to maximize the number of viral particles produced by each cell. Consequently it will be necessary to monitor and control reaction conditions such as temperature, dissolved oxygen, pH, etc. Commercially available bioreactors such as the CelliGen Plus Bioreactor (commercially available from New Brunswick Scientific, Inc. 44 Talmadge Road, Edison, NJ) have provisions for monitoring and maintaining such parameters. Optimization of infection and culture conditions will vary somewhat, however, conditions for the efficient replication and production of virus may be achieved by those of skill in the art taking into considerations the known properties of the producer cell line, properties of the virus, type of bioreactor, etc. When 293 cells are employed as the producer cell line, oxygen concentration is preferably maintained from approximately 50% to approximately 120% dissolved oxygen, preferably 100% dissolved oxygen. When the concentration of viral particles (as determined by conventional methods such as HPLC using a Resource Q column) begins to plateau, the reactor is harvested.

The term "harvesting" means the collection of the cells containing the recombinant adenovirus from the media. This may be achieved by conventional methods such as differential centrifugation or chromatographic means. At this stage, the harvested cells may be stored or further processed by lysis and purification to isolate the recombinant virus. For storage, the harvested cells should be buffered at or about physiological pH and frozen at -70C.

The term "lysis" refers to the rupture of the producer cells. Lysis may be achieved by a variety of means well known in the art. When it is desired to isolate the viral particles from the producer cells, the cells are lysed, using a variety of means well known in the art. For example, mammalian cells may be lysed under low pressure (100-200 psi differential pressure) conditions or conventional freeze thaw methods. Exogenous free DNA/RNA is removed by degradation with DNAse/RNAse.

The term "purifying" means the isolation of a substanially pure population of recombinant virus particles from the lysed producer cells. Conventional purification techniques such as chromatographic or differential density gradient centrifugation methods may be employed. In the preferred practice of the invention, the virus is purified by column chromatography in substantial accordance with the process of Huyghe *et al.* (1995) *Human Gene Therapy* 6*:* 1403-1416 as described in co-pending United States Patent application Serial No. 08/400,793 filed March 7, 1995.

Additional methods and procedures to optimize production of the recombinant adenoviruses of the present invention are described in co-pending United States Patent Application Serial No. 09/073,076, filed May 4, 1998 entitled Viral Production Process.

### EXAMPLES

The following examples provide the methodology and results of experiments demonstrating the construction of particular recombinant adenoviral and plasmid vectors. It will be apparent to those of skill in the art to which the invention pertains, the present invention may be embodied in forms other than those specifically disclosed in these examples, without departing from the spirit or essential characteristics of the invention. The particular embodiments of the invention described below, are therefore to be considered as illustrative and not restrictive. In the following examples, "g" means grams, "ml" means milliliters, "mol" means moles, "°C" means degrees Centigrade, "min." means minutes, "FBS" means fetal bovine serum, and "PN" refers to number of particles of recombinant virus.

### Example 1. Plasmid Constructions

p800luc, a plasmid encoding luciferase under the control of 800 bp PAI promoter was obtained from Dr. David Luskutoff (Scripps Institute, LaJolla, California). Plasmid pCTMIE-E2F-Rb that contains a CMV promoter followed by adenovirus-5 tripartite leader sequence and a SV40 enhancer upstream of the coding sequence for E2F-RB fusion protein was provided by Doug Antelman (Canji).

### Example 2. Construction luciferase plasmids with TGF-β-responsive promoters

### A. PAI-luciferease plasmid:

Sequences of all the fragments are shown in 5'-3' direction A 749 bp fragment flanked with SacI and XhoI sites at 5' and 3' ends respectively, and SV40 TATA box instead of native TATA box within the promoter, was amplified by PCR using p800luc as the template and primers AGT CGA GCT CCA ACC TCA GCC AGA and GAT CCT CGA GCT CCT CTG TGG GCC ACT GCC TCC TCA TAA ATA CC (containing SV40 TATA box ). The resulting PCR product was digested with SacI and XhoI and ligated to a fragment obtained after digesting PGL3-basic, a luciferase construct with no promoter (Promega), to obtain PAI-luciferase.

### B. SRE-luciferase plasmid:

The following oligonucleotides, GG TAT TTA TGA GGA GGC AGT GGC CCA CAG AGG AGC TCG AGG ATC and GAT CCT CGA GCT CCT CTG TGG GCC ACT GCC TCC TCA TAA ATA CC were annealed. The annealed product was digested with XhoI and ligated to PGL3-basic digested with MluI, blunt-ended with Klenow and digested with XhoI to obtain pSVT-luc (a luciferase construct with SV40 TATA box). Oligonucletides containing Smad4/DPC4 binding sites, CGT CTA GAC GTC TAG ACG TCT AGA CGT CTA GAC TGT AC and AGT CTA GAC GTC TAG ACG TCT AGA CGT CTA GAC GGT AC were annealed and ligated to pSVT-luciferase digested with KpnI to obtain SRE-luciferase plasmid.

### Example 3. Construction luciferase plasmids with p53-responsive promoters.

### A. RGC-luciferase plasmid:

Two complimentary 5'-phosphorylated oligonucleotides containing p53 binding sites from the ribosomal gene cluster, AG AAA AGG CAA GGC CAG GCA AGT CCA GGC AAC TCG TGG TAC and CA CGA GTT GCC TGG ACT TGC CTG GCC TTG CCT TTT CTG TAC were annealed. The annealed fragment was ligated to pSVT-luciferase digested with KpnI to obtain RGC-luciferase plasmid.

### B. p53CON-luciferase plasmid:

Two complimentary 5'-phosphorylated oligonucleotides containing consensus p53 binding sites (p53CON), CT CGA CGG ACA TGC CCG GGC ATG TCC TCG ACG GAC ATG CCC GGG CAT GTC CTG TAC and AG GAC ATG CCC GGG CAT GTC CGT CGA GGA CAT GCC CGG GCA TGT CCG TCG AGG TAC were annealed. The annealed fragment was ligated to pSVT-luciferase digested with KpnI to obtain p53CON-luciferase plasmid.

### C. PAI-E2F-Rb plasmid:

The sequence containing the CMV promoter followed by adenovirus-5 tripartite leader sequence and a SV40 enhancer upstream of the coding sequence for E2F-RB fusion protein in plasmid pCTMIE-E2F-Rb was excised by digesting with BglI and XbaI and the resulting fragment was ligated to obtain the promoter-less E2F-RB plasmid. A fragment containing modified PAI promoter was excised from the plasmid PAI-luciferase by digesting with SacI and XhoI and was blunt ended by treating with Klenow. This fragment was ligated to the promoter-less E2F-RB plasmid that was digested with EcoRI and treated with Klenow fragment of DNA polymerase I to obtain PAI-E2F-RB plasmid.

### Example 4. Generation of recombinant adenoviruses

A transfer plasmid containing Ad5 sequence with 3-kb deletion in the E3 region, pNEBΔE3, was constructed by cloning a 7.4 kb SnaBI fragment from pBHG11 (26676 to 34140) to pNEB193 (plasmid from NEB) treated with BamHI, AccI and Klenow. A multiple cloning site was introduced to the above plasmid by annealing 5-phosphorylated oligonucleotides, AAA TAC GTA ATG CAT TCT AGA GCG GCC GCT CGC GAG GAT CCT TAA T and TAA GGA TCC TCG CGA GCG GCC GCT CTA GAA TGC ATT ACG TAT TTA T and introducing the annealed fragment by ligating to the PacI digested pNEBΔE3 to obtain pNEBΔE3(MCS) plasmid.

Transfer plasmid to generate a recombinant adenovirus encoding E2F-Rb under the control of PAI-promoter and enhanced green fluorescent protein (GFP) under the control of CMV promoter was constructed as follows. An intermediate vector pABS.4-E2F-Rb was prepared by ligating a fragment containing PAI promoter and E2F-Rb coding sequence prepared by digesting PAI-E2F-Rb with NacI and XbaI and ligating to pABS.4 plasmid (Microbix) fragment prepared by digesting with KpnI, treating with Klenow and redigesting with Xbal. A fragment containing PAI promoter, E2F-Rb coding sequence and Kanamycin gene was then excised from the plasmid PABS.4-E2F-Rb by digesting with PacI, treated with Klenow and ligated to the plasmid fragment obtained by digesting pNEBΔE3 plasmid with PacI and treating with Klenow to obtain the plasmid pNEBΔE3-PAI-E2F-Rb, with no PacI site. The kanamycin gene in this plasmid was replaced with CMV promoter operably linked to green fluorescent protein (GFP) gene by digesting pNEBΔE3-PAI-E2F-Rb with SwaI and ligating it to a fragment isolated from pEGFP-N (Clontech) by digesting with AfIII and SspI and treating with Klenow, to obtain pΔE3-PAI-E2F-Rb-GFP plasmid.

Transfer plasmids to generate recombinant adenoviruses encoding E2F-Rb under the control of a TGF-β responsive promoter with SRE and enhanced green fluorescent protein (GFP) under the control of CMV promoter were constructed as follows. E2F-Rb coding sequence was introduced into the plasmid pNEBΔE3(MCS) to obtain the plasmid pNEBΔE3-E2F-Rb by ligating a fragment isolated by digesting pNEBΔE3(MCS) with XbaI and NruI and a fragment generated by digesting pCTMIE-E2F-Rb with XbaI and Nae I. A TGF-β-responsive promoter containing SRE was introduced to the above plasmid by amplifying this sequence by PCR using SRE-luciferase as the template and phosphorylated primers, GTA AGG TGC CAG AAC ATT TCT C and GAT AAC TAG TGC TCC TCT GTG GGC CAC T. The resulting PCR product was digested with SpeI and ligated to SnaBI and XbaI digested pNEBΔE3-E2F-Rb to obtain pΔE3-DPC-E2F-Rb.

Transfer plasmids to generate recombinant adenoviruses encoding E2F-Rb under the control of p53 responsive promoters and enhanced green fluorescent protein (GFP) under the control of CMV promoter were constructed as follows. p53-responsive promoter containing p53-binding sites from ribosomal gene cluster or p53 consensus site (p53CON) was introduced to the plasmid pNEBΔE3-E2F-Rb by amplifying the promoter sequence by PCR using RGC-luciferase or p53CON-luciferase as the template and phosphorylated primers, GTA AGG TGC CAG AAC ATT TCT C and GAT ATC TAG ACG TCC TCT GTG GGC CAC T. The resulting PCR products were digested with XbaI and ligated to SnaBI and XbaI digested pNEBΔE3-E2F-Rb to obtain pAE3-RGC-E2F-Rb and pdelataE3-PCON-E2F-Rb.

### Example 5. Homologous recombination to generate recombinant adenoviruses.

Recombinant adenoviruses, PAI-Ad, SRE-Ad, RGC-Ad and CON-Ad were generated by performing homologous recombination in bacteria as described (Chartier *et al.* (1996) J. Virol. 70:4805-4810). The transfer plasmids were digested with AscI to obtain the fragments containing E2F-Rb under the control of pathway-responsive promoter and GFP under the control of CMV promoter flanked by Ad5 sequences. The resulting fragment was cotransformed into BJ 5183 bacterial strain with a fragment obtained by digesting PTG4609 (available from Transgene, Inc. and described in Chartier, *et al.* (1996) J. Virol. 70:4805-4810) with BstBI and SpeI. The resulting bacterial colonies were screened for the presence of desired recombinant Ad5 infectious plasmids, pPAI-GFP-Ad, pSRE-GFP-Ad, pRGC-GFP-Ad and pCON-GFP-Ad. These infectious plasmids were then linearized by digesting with PacI and purified by phenol-choloroform extraction and ethanol precipitatation and used for transfection of 293 cells.

Transfection was performed using Superfect (Qiagen) according to manufacturer's instructions. 2.5 µg of linearized plasmids were used for transfection of 250,000 cells grown in 6-well plates with 12 µl Superfect/well. After 8 days cytopathic effect due to virus production was observed. Cells were harvested along with the culture supernatants and subjected to three rounds of freeze-thaw cycles. Recombinant viruses in the resulting lysates were amplified by reinfecting 293 cells.

### Example 6. Cell lines

All cell lines used in this study were obtained from ATCC (Rockville, MD) and grown as monolayer cultures maintained at 37°C in a CO₂ incubator. 293, Hep3B, MRC9, A549, Panc1, U87, Caco2, MCF-7 and WIDR and were maintained in Dulbecco's modified Eagle's medium supplemented with 10% fetal bovine serum. MDA-MB 468 cells were cultured in Ham's supplemented with 10% fetal bovine serum, whereas MiaPaca-2 cells were grown in DMEM supplemented with 10% fetal bovine serum and 2.5% horse serum.

### Example 7. Transfection

Cells were plated either in 6-well plates (250,000 cells/well) or in 24-well plates (62,500 cells/well) and allowed to attach overnight. Transfections were then carried out using calcium phosphate for 293 cells as described and with Superfect (Qiagen) for other cells according to manufacturer's instructions.

### Example 8. Reporter/luciferase assays

Cells were transfected with 1.5 µg reporter plasmid and 1.0 µg of the inhibitors. 48 h post-transfection lysates were prepared by adding and incubating at room temperature for 10 min in 1X reporter lysis buffer (Promega). Luciferase activity was determined using Top Count (Packard) and an assay kit from Packard according to the instructions from Packard.

### Example 9. Assays to measure virus-mediated cytopathic effect.

Cells were infected with indicated recombinant or wild-type adenoviruses and stained with 0.5% crystal violet prepared in 20% ethanol 6 days post-infection in substantial accordance with the procedure described in Bischoff, *et al.* (1996) Science 274:373-376.

### Example 10 Construction of cU3EE and cT1LT Viruses

The MLP promoter sequence was amplified by PCR using the primers GAT CCG ATC GAT AGC GCG TAA TAT TTG TCT AGG GC and GAT CTT AAT TAA ATG GCA GTG ACC CGG AAG using Ad5 DNA such as in the plasmid pFG140 (Microbix) as the template. The MLP PCR product was then cloned at the PacI site in the plasmid pdelataE3-PCON-E2F-Rb to obtain pdelataE3-PCON-E2F-Rb-MLP. Adenovirus E3 10.5K protein coding sequence was amplified by PCR using the primers, GCG ACC CAC CCT AAC AGA and GAT CGG ATC CAA AGC GCA ACA AGG GTC A and the resulting PCR product was cloned at the Xho I site in the plasmid pCDNA3.1 (Invitrogen) to obtain pCDNA3-10.5 plasmid. Adenovirus E3 10.5K protein coding sequence was then excised from pCDNA3-10.5 by digesting with DraIII and XbaI followed by Klenow treatement and was ligated to PacI and Klenow treated pdelataE3-PCON-E2F-Rb-MLP to obtain pdelataE3-PCON-E2F-Rb-MLP-10.5K plasmid.

Recombinant adenoviruses cU3EE and cT1LT were generated by performing homologous recombination in bacteria as described (Chartier et al. ,1996, J. Virol. 70:4805-4810). The transfer plasmid was digested with AscI to obtain the fragments containing E2F-Rb under the control of p53CON sequence and E310.5K under the control of MLP promoter flanked by Ad5 sequences. The resulting fragment was cotransformed into BJ 5283 bacterial strain with a fragment obtained by digesting PTG4609 (Transgene) with BstBI and SpeI. The resulting bacterial colonies were screened for the presence of desired recombinant Ad5 infectious plasmid pCEMD. The Ad5 infectious plasmid p01/CEMD was obtained by following a similar protocol but by using a derivative of PTG4609 (Transgene) in which wild-type E1A sequence was replaced with a sequence containing E1A with 01 mutation. These infectious plasmids were then linearized by digesting with PacI, purified by phenol-choloroform extraction and ethanol precipitatation and used for transfection of 293 cells.

Transfection of plasmids pCEMD and p01/CEMD was performed to generate recombinant viruses cU3EE and cT1LT respectively using Superfect (Qiagen) according to manufacturer's instructions. 2.5 µg of linearized plasmids were used for transfection of 500,000 cells grown in 6-well plates with 12 µl Superfect/well. After 8 days cytopathic effect due to virus production was observed. Cells were harvested along with the culture supernatants and subjected to three rounds of freeze-thaw cycles. Recombinant viruses in the resulting lysates were amplified by reinfecting 293 cells.

## Claims

1. A selectively replicating recombinant viral vector comprising a pathway-responsive promoter operably linked to a repressor of viral replication, wherein the vector selectively replicates in neoplastic cells possessing a defect in the pathway to which the promoter is responsive.

2. The vector of claim 1, wherein the neoplastic cells are tumor cells.

3. The vector of claims 1-2 wherein the pathway-responsive promoter is selected from the group consisting of a p53 pathway-responsive promoter, an Rb pathway responsive promoter and a TGF-beta pathway-responsive promoter.

4. The vector of claim 3 wherein the virus is derived from the genus adenoviridiae.

5. The vector of claim 4 wherein the repressor of viral replication is E2F-RB.

6. The vector of claims 1-2, further comprising a transgene expression cassette.

7. The vector of claim 6, wherein the transgene expression cassette comprises a pro-apoptotic gene operably linked to a promoter.

8. The vector of claim 7 wherein the pro-apoptotic gene is Ad5 E3-10.5K.

9. The vector of claim 8 wherein the pathway responsive promoter is a p53 pathway responsive promoter.

10. The vector of claim 9 wherein the p53 pathway responsive promoter is p53CON which has the sequence CT CGA CGG ACA TGC CCG GGC ATG TCC TCG ACG GAC ATG CCC GGG CAT GTC CTG TAC or AG GAC ATG CCC GGG CAT GTC CGT CGA GGA CAT GCC CGG GCA TGT CCG TCG AGG TAC.

11. The vector of claim 10 wherein the promoter component of the transgene expression cassette is a temporal promoter.

12. The vector of claim 11 wherein the temporal promoter is the major late promoter (MLP).

13. The vector of claim 12 further comprising a deletion in the adenoviral E1a coding region so as to eliminate p300 binding.

14. The vector of claim 13 wherein the deletion in the adenoviral E1a coding region comprises a deletion of amino acids 4-25 of the E1a 289R protein.

15. A pharmaceutical formulation comprising as an active ingredient a selectively replicating recombinant vector as claimed in any one of claims 1-14 associated with one or more pharmaceutically acceptable carriers therefor.

16. A method of killing a cell with a pathway defect by contacting said cell *ex vivo* with a selectively replicating recombinant virus as claimed in any one of claims 1-14.

17. Use of a selectively replicating viral vector as claimed in any one of claims 1-14 for the preparation of a pharmaceutical composition for killing a cell with a pathway defect.

18. Use according to claim 17, wherein the pharmaceutical composition is used for treating cancer.

19. An isolated cell transformed with selectively replicating recombinant virus comprising a pathway-responsive promoter operably linked to a repressor of viral replication.

20. A process for producing a selectively replicating viral vector as claimed in any one of claims 1-14 said process comprising infecting a population of producer cells with a sample of said vector, culturing said producer cell line under conditions to permit replication of said vector in said producer cells and isolating the vector from the producer cells.

## Patentansprüche

1. Ein sich selektiv replizierender, rekombinanter, viraler Vektor, der einen auf einen Syntheseweg ("pathway") ansprechenden Promotor umfaßt, der in funktionsfähiger Weise an einen Repressor der viralen Replikation gekoppelt ist, wobei sich der Vektor selektiv in neoplastischen Zellen repliziert, die einen Defekt in dem Syntheseweg aufweisen, auf den der Promotor anspricht.

2. Vektor gemäß Anspruch 1, bei dem die neoplastischen Zellen Tumorzellen sind.

3. Vektor gemäß den Ansprüchen 1-2, bei dem der auf einen Syntheseweg ansprechende Promotor ausgewählt ist aus der Gruppe bestehend aus einem auf einen p53-Syntheseweg ansprechenden Promotor, einem auf einen Rb-Syntheseweg ansprechenden Promotor und einem auf einen TGF-beta-Syntheseweg ansprechenden Promotor.

4. Vektor gemäß Anspruch 3, bei dem das Virus aus der Gattung der Adenoviridiae abgeleitet ist.

5. Vektor gemäß Anspruch 4, bei dem E2F-RB der Repressor der viralen Replikation ist.

6. Vektor gemäß den Ansprüchen 1-2, der weiterhin eine Transgen-Expressionskassette umfaßt.

7. Vektor gemäß Anspruch 6, bei dem die Transgen-Expressionskassette ein pro-apoptotisches Gen in funktionsfähiger Weise an einen Promotor gekoppelt umfaßt.

8. Vektor gemäß Anspruch 7, bei dem Ad5 E3-10.5K das proapoptotische Gen ist.

9. Vektor gemäß Anspruch 8, bei dem der auf einen Syntheseweg ansprechende Promotor ein auf einen p53-Syntheseweg ansprechender Promotor ist.

10. Vektor gemäß Anspruch 9, bei dem der auf einen p53-Syntheseweg ansprechende Promotor p53CON ist, der die Sequenz CT CGA CGG ACA TGC CCG GGC ATG TCC TCG ACG GAC ATG CCC GGG CAT GTC CTG TAC oder AG GAC ATG CCC GGG CAT GTC CGT CGA GGA CAT GCC CGG GCA TGT CCG TCG AGG TAC aufweist.

11. Vektor gemäß Anspruch 10, bei dem der Promotorbestandteil der Transgen-Expressionskassette ein temporärer Promotor ist.

12. Vektor gemäß Anspruch 11, bei dem der temporäre Promotor der "major late"-Promotor (MLP) ist.

13. Vektor gemäß Anspruch 12, der weiterhin eine Deletion in dem für adenovirales Ela kodierenden Bereich umfaßt, um p300-Bindung auszuschließen.

14. Vektor gemäß Anspruch 13, bei dem die Deletion in dem für adenovirales E1a kodierenden Bereich eine Deletion der Aminosäuren 4-25 des E1a 289R-Proteins umfaßt.

15. Pharmazeutische Formulierung, die als Wirkstoff einen sich selektiv replizierenden, rekombinanten Vektor, wie er in einem der Ansprüche 1-14 beansprucht ist, in Verbindung mit einem oder mehreren pharmazeutisch verträglichen Träger(n) für diesen umfaßt.

16. Verfahren zum Abtöten einer Zelle mit einem Syntheseweg-Defekt durch in Kontakt bringen der Zelle *ex vivo* mit einem sich selektiv replizierenden, rekombinanten Virus, wie es in einem der Ansprüche 1-14 beansprucht ist.

17. Verwendung eines sich selektiv replizierenden, viralen Vektors gemäß einem der Ansprüche 1-14 zur Herstellung einer pharmazeutischen Formulierung zum Abtöten einer Zelle mit einem Syntheseweg-Defekt.

18. Verwendung gemäß Anspruch 17, bei der die pharmazeutische Formulierung zum Behandeln von Krebs verwendet wird.

19. Eine isolierte Zelle, die mit einem sich selektiv replizierenden, rekombinanten Virus transformiert wurde, das einen auf einen Syntheseweg ansprechenden Promotor umfaßt, der in funktionsfähiger Weise an einen Repressor der viralen Replikation gekoppelt ist.

20. Verfahren zum Herstellen eines sich selektiv replizierenden, viralen Vektors, wie er in einem der Ansprüche 1-14 beansprucht ist, wobei das Verfahren das Infizieren einer Population von Producerzellen mit einer Probe des Vektors, das Kultivieren der Producer-Zellinie unter Bedingungen, welche die Replikation des Vektors ermöglichen, und das Isolieren des Vektors aus den Producerzellen umfaßt.

## Revendications

1. Vecteur viral recombinant à réplication sélective comprenant un promoteur sensible à une voie lié opérationnellement à un répresseur de la réplication virale, où le vecteur se réplique sélectivement dans des cellules néoplasiques possédant un défaut dans la voie à laquelle est sensible le promoteur.

2. Vecteur selon la revendication 1 où les cellules néoplasiques sont des cellules tumorales.

3. Vecteur selon les revendications 1 et 2, dans lequel le promoteur sensible à une voie est choisi dans le groupe constitué par un promoteur sensible à la voie de p53, un promoteur sensible à la voie de Rb et un promoteur sensible à la voie du TGF-béta.

4. Vecteur selon la revendication 3, où le virus est dérivé du genre des adenoviridae.

5. Vecteur selon la revendication 4, où le répresseur de la réplication virale est E2F-RB.

6. Vecteur selon les revendications 1 et 2, comprenant en outre une cassette d'expression de transgène.

7. Vecteur selon la revendication 6, dans lequel la cassette d'expression de transgène comprend un gène pro-apoptotique lié opérationnellement à un promoteur.

8. Vecteur selon la revendication 7, dans lequel le gène pro-apoptotique est Ad5 E3-10.5K.

9. Vecteur selon la revendication 8, dans lequel le promoteur sensible à une voie est un promoteur sensible à la voie de p53.

10. Vecteur selon la revendication 9, dans lequel le promoteur sensible à la voie de p53 est le p53CON qui a la séquence CT CGA CGG ACA TGC CCG GGC ATG TCC TCG ACG GAC ATG CCC GGG CAT GTC CTG TAC ou AG GAC ATG CCC GGG CAT GTC CGT CGA GGA CAT GCC CGG GCA TGT CCG TCG AGG TAC.

11. Vecteur selon la revendication 10, dans lequel le composant promoteur de la cassette d'expression de transgène est un promoteur temporel.

12. Vecteur selon la revendication 11, dans lequel le promoteur temporel est le promoteur tardif majeur (PTM ou MLP).

13. Vecteur selon la revendication 12, comprenant en outre une délétion dans la région adénovirale codant E1a de manière à éliminer la liaison à p300.

14. Vecteur selon la revendication 13, dans lequel la délétion dans la région adénovirale codant E1a comprend une délétion des acide aminés 4 à 25 de la protéine E1a 289R.

15. Formulation pharmaceutique comprenant comme ingrédient actif un vecteur recombinant à réplication sélective tel que revendiqué dans l'une quelconque des revendications 1 à 14 associé avec un ou plusieurs véhicules pharmaceutiquement acceptables pour celui-ci.

16. Procédé pour tuer une cellule ayant un défaut dans une voie en mettant en contact *ex vivo* ladite cellule avec un virus recombinant à réplication sélective tel que revendiqué dans l'une quelconque des revendications 1 à 14.

17. Utilisation d'un vecteur viral à réplication sélective selon l'une quelconque des revendications 1 à 14 pour la préparation d'une composition pharmaceutique afin de tuer une cellule ayant un défaut dans une voie.

18. Utilisation selon la revendication 17, dans laquelle la composition pharmaceutique est utilisée pour traiter le cancer.

19. Cellule isolée transformée avec un virus recombinant à réplication sélective comprenant un promoteur sensible à une voie lié opérationnellement à un répresseur de réplication virale.

20. Procédé de production d'un vecteur viral à réplication sélective selon l'une quelconque des revendications 1 à 14, ledit procédé comprenant l'infection d'une population de cellules productrices avec un échantillon dudit vecteur, la culture de ladite lignée cellulaire productrice dans les conditions permettant la réplication dudit vecteur dans lesdites cellules productrices et l'isolement du vecteur des cellules productrices.


### SEQUENCE LISTING

<110> Canji, Inc.
<120> Selectively Replicating Viral Vectors
<130> CJ-0926 K Seq PCT
<140> PCT/US 99/21452
   <141> 1999-10-14
<160> 18
<170> PatentIn Ver. 2.1
<210> 1
   <211> 24
   <212> DNA
   <213> Artificial Sequence
<220>
   <223> Description of Artificial Sequence: PCR Primer
<400> 1
   agtcgagctc caacctcagg caga 24
<210> 2
   <211> 42
   <212> DNA
   <213> Artificial Sequence
<220>
   <223> Description of Artificial Sequence: PCR Primer
<400> 2
   gatccgcgag ctccgctgtg ggccactgcc tcctcataaa ta 42
<210> 3
   <211> 44
   <212> DNA
   <213> Artificial Sequence
<220>
   <223> Description of Artificial Sequence: Linker to Insert SV-40 TATA Box
<400> 3
   ggtatttatg aggaggcagt ggcccacaga ggagctcgag gatc 44
<210> 4
   <211> 44
   <212> DNA
   <213> Artificial Sequence
<220>
   <223> Description of Artificial Sequence: Linker to Insert SV-40 TATA Box
<400> 4
   gatcctcgag ctcctctgtg ggccactgcc tcctcataaa tacc 44
<210> 5
   <211> 38
   <212> DNA
   <213> Artificial Sequence
<220>
   <223> Description of Artificial Sequence: PCR Primers for SMAD4/DPC4 Binding Sites
<400> 5
   cgtctagacg tctagacgtc tagacgtcta gactgtac 38
<210> 6
   <211> 38
   <212> DNA
   <213> Artificial Sequence
<220>
   <223> Description of Artificial Sequence: PCR Primers for SMAD4/DPC4 Binding Sites
<400> 6
   agtctagacg tctagacttc tagacgtcta gacggtac 38
<210> 7
   <211> 41
   <212> DNA
   <213> Artificial Sequence
<220>
   <223> Description of Artificial Sequence: P53 Binding Sites from ribosomal gene cluster
<400> 7
   agaaaaggca aggccaggca agtccaggca cctcgtggta c 41
<210> 8
   <211> 41
   <212> DNA
   <213> Artificial Sequence
<220>
   <223> Description of Artificial Sequence: P53 Binding Sites from ribosomal gene cluster
<400> 8
   cacgagttgc ctggacttgc ctggccttgc cttttctgta c 41
<210> 9
   <211> 56
   <212> DNA
   <213> Artificial Sequence
<220>
   <223> Description of Artificial Sequence: PCR Primers for p53 Binding Domains
<400> 9
   ctcgacggac atgcccgggc atgtcctcga cggacatgcc cgggcatgtc ctgtac 56
<210> 10
   <211> 56
   <212> DNA
   <213> Artificial Sequence
<220>
   <223> Description of Artificial Sequence: PCR Primers for p53 Binding Domains
<400> 10
   aggacatgcc cgggcatgtc cgtcgaggac atgcccgggc atgtcggtcg aggtac 56
<210> 11
   <211> 46
   <212> DNA
   <213> Artificial Sequence
<220>
   <223> Description of Artificial Sequence: Multiple Cloning Site
<400> 11
   aaatacgtaa tgcattctag agcggccgct cgcgaggatc cttaat 46
<210> 12
   <211> 46
   <212> DNA
   <213> Artificial Sequence
<220>
   <223> Description of Artificial Sequence: Multiple Cloning Site
<400> 12
   taaggatcct cgcgagcggc cgctctagaa tgcattacgt atttat 46
<210> 13
   <211> 22
   <212> DNA
   <213> Artificial Sequence
<220>
   <223> Description of Artificial Sequence: PCR Primers
<400> 13
   gtaaggtgcc agaacatttc tc 22
<210> 14
   <211> 28
   <212> DNA
   <213> Artificial Sequence
<220>
   <223> Description of Artificial Sequence: PCR Primers
<400> 14
   gatacctagt gctcctctgt gggccact 28
<210> 15
   <211> 35
   <212> DNA
   <213> Artificial Sequence
<220>
   <223> Description of Artificial Sequence: PCR Primers for MLP Promoter
<400> 15
   gatccgatcg atagcgcgta atatttgtct agggc 35
<210> 16
   <211> 30
   <212> DNA
   <213> Artificial Sequence
<220>
   <223> Description of Artificial Sequence: PCR Primers for MLP Promoter
<400> 16
   gatcttaatt aaatggcagt gacccggaag 30
<210> 17
   <211> 18
   <212> DNA
   <213> Artificial Sequence
<220>
   <223> Description of Artificial Sequence: PCR Primer for Adenovirus E3 10.5K Protein
<400> 17
   gcgacccacc ctaacaga 18
<210> 18
   <211> 28
   <212> DNA
   <213> Artificial Sequence
<220>
   <223> Description of Artificial Sequence: PCR Primer for Adenovirus E3 10.5K Protein
<400> 18
   gatcggatcc aaagcgcaac aagggtca 28